# EUROPÄISCHE PATENTSCHRIFT

(11) **EP 3 598 947 B1**
(45) Veröffentlichungstag und Bekanntmachung des Hinweises auf die Patenterteilung: **26.08.2020**
(21) Anmeldenummer: 18185766.5
(22) Anmeldetag: 26.07.2018
(51) Int. Cl.: A61B 6/00, A61B 6/10, A61B 6/03

(54) **RÖNTGENANORDNUNG MIT MEHREREN RÖNTGENEINRICHTUNGEN UND VERFAHREN ZUM BETRIEB EINER RÖNTGENANORDNUNG**
X-RAY ARRANGEMENT WITH MULTIPLE X-RAY DEVICES AND METHOD FOR OPERATING AN X-RAY ARRANGEMENT
SYSTÈME À RAYONS X DOTÉ D'UNE PLURALITÉ DE DISPOSITIFS À RAYONS X ET PROCÉDÉ DE FONCTIONNEMENT D'UN SYSTÈME À RAYONS X

(43) Veröffentlichungstag der Anmeldung: 29.01.2020
(73) Patentinhaber: Siemens Healthcare GmbH, 91052 Erlangen (DE)
(72) Erfinder: Pickert, Nils, 91056 Erlangen (DE); Dressler, Christian, 91074 Herzogenaurach (DE); Bittner, Maik, 91080 Marloffstein (DE)

(56) Entgegenhaltungen:
- EP-A1- 3 267 428
- DE-A1-102013 219 193
- US-A1- 2002 150 214
- US-A1- 2015 327 833
- US-A1- 2016 345 920
- US-A1- 2016 374 640

## Beschreibung

Die Erfindung betrifft eine Röntgenanordnung, umfassend wenigstens zwei Röntgeneinrichtungen mit jeweils einer Steuereinrichtung und einer einen Röntgenstrahler und einen Röntgendetektor aufweisenden Aufnahmeanordnung, wobei wenigstens eine der Röntgeneinrichtungen eine mobile Röntgeneinrichtung mit einem mobilen, die Aufnahmeanordnung tragenden Träger ist. Daneben betrifft die Erfindung ein Verfahren zum Betrieb einer solchen Röntgenanordnung.

Neben fest installierten Röntgeneinrichtungen wurden im Stand der Technik bereits mobile Röntgeneinrichtungen, wie z.B. aus der US 2016/0345920 A1 bekannt, vorgeschlagen, beispielsweise sogenannte mobile C-Bögen, die mithin einen Träger aufweisen, der mit einer Mobilitätseinrichtung, beispielsweise Rädern, versehen ist und so frei im Raum bewegt und positioniert werden kann. Derartige mobile Röntgeneinrichtungen nähern sich in ihrer Leistungsfähigkeit sowohl bei der 2D-Bildgebung als auch bei der 3D-Bildgebung fest installierten Röntgeneinrichtungen immer mehr an und können heute, insbesondere wenn der Mobilitätseinrichtung auch eine Antriebseinrichtung zugeordnet ist, auch selbstfahrend ausgestaltet werden, so dass mit hoher Genauigkeit an einen Patiententisch oder dergleichen herangefahren werden kann bzw. auch eine hochverlässliche Repositionierung ermöglicht ist. Der Hauptvorteil solcher mobiler Röntgeneinrichtungen ist die gesteigerte Flexibilität, da die mobile Röntgeneinrichtung bei Bedarf aus dem Raum entfernt werden kann und/oder in eine weniger störende Position verbracht werden kann, was insbesondere bei an dem Patienten vorzunehmenden Untersuchungen und/oder Eingriffen nützlich ist.

Bei fest installierten Röntgeneinrichtungen, beispielsweise angiographischen Systemen mit ein oder zwei fest installierten C-Bögen, ist die Raumnutzung für bestimmte Anwendungen festgelegt. Insbesondere kann bei der Verwendung einer fest installierten Biplan-Röntgeneinrichtung im Betrieb mit nur einer der zwei Aufnahmeanordnungen (Einzel-Ebenen-Betrieb) auch bei Positionierung der nicht benötigten Aufnahmeanordnung und ihres C-Bogens in einer Parkposition diese nicht komplett aus dem klinischen Bereich entfernt werden, so dass die Arbeit des Personals im Untersuchungs- bzw. Behandlungsraum behindert werden kann. Vorgeschlagen wurde im Stand der Technik auch bereits, eine fest installierte Biplan-Angiographie-Röntgeneinrichtung mit einer sogenannten Sliding Gantry-CT-Einrichtung zu kombinieren, wobei ein Patiententisch um 90° drehbar gestaltet wird, um zwischen diesen Modalitäten zu wechseln. Dies ist notwendig, nachdem der fest am Boden montierte C-Bogen der Biplan-Angiographie-Röntgeneinrichtung nicht aus der Patientenachse herausbewegt werden kann, so dass die Sliding Gantry-CT-Röntgeneinrichtung um 90° gedreht zu der Biplan-Angiographie-Röntgeneinrichtung positioniert werden muss. Eine Sliding Gantry-CT-Röntgeneinrichtung zeichnet sich dadurch aus, dass die Gantry auf Schienen geführt bewegbar ist, beispielsweise zwischen zwei benachbarten Räumen.

Mithin ist insbesondere bei Biplan-Röntgeneinrichtungen ein erhöhter Platzbedarf gegeben, der zudem zu einem unkomfortablen Workflow bei kombinierten Biplan-Angiographie-CT-Anlagen führt.

Allerdings muss auch darauf hingewiesen werden, dass die zeitgleiche Bildgebung in unterschiedlichen Aufnahmegeometrien ("Mehr-Ebenen-Bildgebung") in vielen Einsatzgebieten auch neben der diagnostischen Angiographie nützlich ist, insbesondere auch bei bildgeführten minimalinvasiven Eingriffen an einem Patienten. Beispielsweise kann der Fortschritt von Pedikelschrauben im Rückgrat, der Fortschritt von Nadeln in der Lunge/Leber, die Fortbewegung von Kathetern im Herz/ Gehirn und dergleichen aus verschiedenen Blickwinkeln, insbesondere also verschiedenen Projektionswinkeln, überwacht werden, um Fehlpositionierungen und/oder sogar Verletzungen empfindlicher Anatomie zu vermeiden.

Wie bereits erwähnt, sind fest installierte Biplan-Angiographie-Röntgeneinrichtungen, welche insbesondere zwei C-Bögen mit jeweils zugeordneten Aufnahmeanordnungen aufweisen, im Stand der Technik bereits bekannt und weithin genutzt. Allerdings hat sich gezeigt, dass gerade in Situationen, in denen Kosten eingespart werden sollen oder müssen, aufgrund der bekannt gewordenen mobilen Röntgeneinrichtungen Umgehungslösungen angestrebt werden, bei denen zwei unabhängige mobile Röntgeneinrichtungen mit C-Bögen verwendet werden und manuell als Biplan-System eingesetzt werden. Dabei treten jedoch massive Probleme auf, wenn beispielsweise die C-Bögen mit den daran vorgesehenen Aufnahmeanordnungen gemeinsam mit derselben Winkelgeschwindigkeit und in derselben Richtung gedreht werden sollen. Dabei werden üblicherweise zwei Personen eingesetzt, die auf gesprochene Kommandos die C-Bögen mit gleicher Geschwindigkeit bewegen. Die menschliche Interaktion bringt iterative Korrekturschritte mit sich und garantiert keine perfekt senkrechte Ansicht oder eine festgelegte Winkeldifferenz von 45°. Eine Vielzahl von Korrekturschritten bringt jedoch eine höhere Strahlungsdosis, eine vermehrte Verwendung von Kontrastmitteln, eine Erhöhung der Aufnahmezeitdauer und zusätzliche Personalanforderungen mit sich.

Möglich ist es grundsätzlich auch, nur einen einzigen C-Bogen mit einer Aufnahmeanordnung zu verwenden, wobei dieser C-Bogen mitsamt der Aufnahmeanordnung zwischen wenigstens zwei unterschiedlichen Positionen und Winkeln bewegt werden muss. Diese Alternative ist äußerst aufwendig, verbraucht viel Zeit und ist auch gefährlich, nachdem die Bewegung um den Patienten herum stattfinden muss, welcher mit einer sterilen Abdeckung bedeckt ist und mit anderen Vorrichtungen verbunden ist, beispielsweise Atmungsgeräten, Mikroskopen, Endoskopen, Navigationssystemen und dergleichen. Nachdem Wechsel zwischen Anterior-Posterior-Positionierungen und Lateralpositionierungen sehr oft durchgeführt werden müssen, entsteht ein enormer Aufwand, bei dem es unrealistisch ist, dass ein Arzt dies mehr als ein- bis zweimal pro Prozedur durchführen wird, auch wenn die Fehlerrate unmittelbar beeinflusst wird.

Ein weiteres in diesem Kontext bestehendes Problem ergibt sich, wenn verwendete feste und/oder mobile Röntgeneinrichtungen einen zusätzlichen sogenannten "Monitorwagen" aufweisen, an dem beispielsweise eine Darstellungseinrichtung und eine Bedieneinrichtung für die jeweilige Röntgeneinrichtung vorgesehen sein können. Bei der Verwendung von mehreren Röntgeneinrichtungen sind mehrere derartige Monitorwägen erforderlich, obwohl der Raum innerhalb von Untersuchungs- und/ oder Behandlungsräumen häufig ohnehin eng ist.

Der Erfindung liegt mithin die Aufgabe zugrunde, den Einsatz von mobilen Röntgeneinrichtungen in einer Röntgenanordnung mit wenigstens zwei Röntgeneinrichtungen zu vereinfachen.

Diese Aufgabe wird gelöst durch eine Röntgenanordnung mit den Merkmalen des Anspruchs 1 und ein Verfahren zum Betrieb einer solchen Röntgenanordnung mit den Merkmalen des Anspruchs 15. Vorteilhafte Ausgestaltungen ergeben sich aus den Unteransprüchen.

Bei einer Röntgenanordnung der eingangs genannten Art ist erfindungsgemäß also insbesondere vorgesehen, dass die Röntgeneinrichtungen jeweils eine Kommunikationsschnittstelle zur Herstellung einer Kommunikationsverbindung zwischen den Steuereinrichtungen der Röntgeneinrichtungen und/oder zwischen einer zur Röntgenanordnung gehörigen Zwischeneinrichtung, die selbst eine Steuereinrichtung und wenigstens eine Kommunikationsschnittstelle aufweist, und allen Steuereinrichtungen der Röntgeneinrichtungen aufweisen, wobei die Steuereinrichtung wenigstens einer der wenigstens einen mobilen Röntgeneinrichtung zur Steuerung des Bildaufnahmebetriebs der jeweiligen Röntgeneinrichtung anhand von über die Kommunikationsverbindung von einer anderen Steuereinrichtung erhaltenen Steuerdaten und/oder zur Übermittlung wenigstens eines aufgenommenen Bilddatensatzes an wenigstens eine weitere Steuereinrichtung über die Kommunikationsverbindung ausgebildet ist.

Entsprechend sieht ein Verfahren zum Betrieb einer solchen Röntgenanordnung vor, dass mittels jeweils einer Kommunikationsschnittstelle der Röntgeneinrichtungen eine Kommunikationsverbindung zwischen den Steuereinrichtungen der Röntgeneinrichtungen und/oder zwischen einer zur Röntgenanordnung gehörigen Zwischeneinrichtung, die selbst eine Steuereinrichtung und wenigstens eine Kommunikationsschnittstelle aufweist, und allen Steuereinrichtungen der Röntgeneinrichtung hergestellt wird, wobei die Steuereinrichtung wenigstens einer der wenigstens einen mobilen Röntgeneinrichtung den Bildaufnahmebetrieb der jeweiligen Röntgeneinrichtung anhand von über die Kommunikationsverbindung von einer anderen Steuereinrichtung erhaltenen Steuerdaten steuert und/oder wenigstens einen aufgenommenen Bilddatensatz an wenigstens eine weitere Steuereinrichtung über die Kommunikationsverbindung übermittelt.

Die Erfindung betrifft also im Allgemeinen eine Röntgenanordnung, in der wenigstens zwei, insbesondere genau zwei, Röntgeneinrichtungen zu einem gemeinsamen Zweck nutzbar sein sollen, wobei wenigstens eine dieser Röntgeneinrichtungen mobil ausgebildet ist, das bedeutet, insbesondere eine Mobilitätseinrichtung, beispielsweise umfassend Räder, besitzt, die ein mittels einer entsprechenden Antriebsvorrichtung umgesetztes automatisches und/oder manuelles Bewegen der mobilen Röntgeneinrichtung in dem Raum, in dem die Röntgeneinrichtungen benutzt werden sollen, sowie gegebenenfalls darüber hinaus, erlaubt. Dabei sei darauf hingewiesen, dass im Folgenden die Erfindung häufig im Beispiel von genau zwei Röntgeneinrichtungen in der Röntgenanordnung erläutert werden wird, dies ist jedoch nicht beschränkend, da auch das Zusammenwirken von drei oder mehr Röntgeneinrichtungen, insbesondere zu einer gemeinsamen Bildgebungsaufgabe, mittels der vorliegenden Erfindung deutlich vereinfacht werden kann.

Eine wesentliche Idee der vorliegenden Erfindung ist es also, Kommunikationsschnittstellen vorzusehen, die es den Steuereinrichtungen der Röntgeneinrichtungen erlauben, direkt oder indirekt miteinander und/oder mit einer insbesondere zentralen Steuereinrichtung einer Zwischeneinrichtung, beispielsweise eines Monitorwagens, zu kommunizieren. Dies ermöglicht es insbesondere, die wenigstens eine mobile Röntgeneinrichtung "fernzusteuern", insbesondere also durch das Übersenden von Steuerdaten über die Kommunikationsverbindung einen bestimmten Bildaufnahmebetrieb herzustellen und/oder Ergebnisse des Bildaufnahmebetriebs zur gemeinsamen Verarbeitung mit Ergebnissen anderer Röntgeneinrichtungen an einer gemeinsamen Stelle zu sammeln. Letztendlich erlaubt es das Vorsehen der Kommunikationsschnittstellen und somit der wenigstens einen Kommunikationsverbindung also, die mehreren Röntgeneinrichtungen als ein gemeinsames, zumindest teilweise zusammengefasstes Röntgengerät zu verstehen, was die Nutzung solcher kombinierter Röntgenanordnungen auf verschiedenste Arten, die im Folgenden noch genauer dargestellt werden, deutlich vereinfacht sowie weitere Vorteile zeitigt. Im einfachen Fall von zwei Röntgeneinrichtungen ergeben sich dabei beispielsweise Konstellationen, in denen zwei mobile Röntgeneinrichtungen bzw. eine mobile und eine feste installierte Röntgeneinrichtung unmittelbar über die Kommunikationsverbindung miteinander kommunizieren, um somit den Bildaufnahmebetrieb und/oder die Auswertung/Darstellung der Bilddaten zu koordinieren. In einer anderen Umsetzungsvariante kann auch vorgesehen sein, dass die Röntgeneinrichtungen nicht direkt, sondern über wenigstens eine weitere Einrichtung, insbesondere ein Netzwerk, an dem sich beide Röntgeneinrichtungen anmelden, und/oder ein Krankenhaus- und/oder Kliniksystem kommunizieren. Derartige Netzwerke und/oder Systeme können bereits als Zwischeneinrichtung bzw. eine Zwischeneinrichtung umfassend aufgefasst werden; eine Umsetzungsvariante sieht jedoch auch vor, eine insbesondere ebenso mobile, lokale Zwischeneinrichtung zu verwenden, beispielsweise einen einzelnen Monitorwagen mit einer Darstellungseinrichtung und einer Bedieneinrichtung, von dem aus zentral beide Röntgeneinrichtungen gesteuert werden können, ohne dass weitere Zwischeneinrichtungen, konkret Monitorwägen, erforderlich sind.

Bevorzugt sind die Kommunikationsschnittstellen zur wenigstens teilweise drahtlosen Kommunikation ausgebildet. Dabei können grundsätzlich bekannte drahtlose Kommunikationstechnologien auch im Rahmen der vorliegenden Erfindung eingesetzt werden, beispielsweise mithin die Kommunikationsverbindung gemäß den Bluetooth-Standard und/oder dem WLAN-Standard hergestellt werden. Nachdem entsprechende drahtlose Kommunikationstechnologien weitgehend bekannt sind, sollen sie hier nicht näher dargelegt werden. Denkbar ist es im Rahmen der vorliegenden Erfindung grundsätzlich jedoch auch, eine drahtgebundene Kommunikationsverbindung vorzusehen, beispielsweise also ein Datenkommunikationskabel zwischen den jeweiligen Kommunikationsschnittstellen, die entsprechende Anschlüsse aufweisen, vorzusehen. Dabei kann auch eine wenigstens teilweise drahtgebundene Kommunikation verwendet werden. Beispielsweise kann es sinnvoll sein, allgemein drahtlos zu kommunizieren, jedoch zumindest zeitweise für Synchronisationsaufgaben ein Kabel zu verwenden.

Ferner werden als Röntgeneinrichtungen im Rahmen der vorliegenden Erfindung bevorzugt Röntgeneinrichtungen mit einem C-Bogen verwendet, an dessen Enden sich gegenüberliegend der Röntgenstrahler und der Röntgendetektor der Aufnahmeanordnung angeordnet sind. Bei der wenigstens einen mobilen Röntgeneinrichtung kann mithin vorgesehen sein, dass der Träger für die Aufnahmeanordnung einen C-Bogen umfasst. Die mobile Röntgeneinrichtung kann dann auch als "mobiler C-Bogen" bezeichnet werden. Zumindest als eine der Röntgeneinrichtungen können jedoch auch andere Arten von Röntgeneinrichtungen vorgesehen werden, beispielsweise, worauf im Folgenden noch näher eingegangen werden wird, eine sogenannte Sliding Gantry-CT-Einrichtung.

Allgemein gesagt ist es über die Kommunikationsverbindung möglich, Röntgeneinrichtungen aneinander zu koppeln, so dass insbesondere ein synchronisiertes Mehr-Ebenen-Bildgebungssystem als Röntgengerät entsteht. Dabei wird es zum einen ermöglicht, die wenigstens zwei Röntgeneinrichtungen zentral über eine einzige Bedieneinrichtung zentral zu bedienen, weswegen insbesondere Bedieneinrichtungen anderer Röntgeneinrichtungen auch deaktiviert werden können. Durch die wenigstens eine Kommunikationsverbindung wird ein koordinierter Betrieb der Röntgeneinrichtungen möglich, sowohl was den Bildaufnahmebetrieb als auch den Bildverarbeitungsbetrieb/ Darstellungsbetrieb angeht. Dies reduziert die Fehleranfälligkeit in der Röntgenanordnung enorm und begünstigt qualitativ hochwertige Ergebnisse. Zudem werden deutliche Workflow-Vereinfachungen erreicht. Weisen mehrere Röntgeneinrichtungen eine Darstellungseinrichtung zur Wiedergabe aufgenommener Bilddatensätze auf, so können aufgrund der Kommunikationsverbindungen die jeweiligen Darstellungseinrichtungen genutzt werden, um auch Bilddatensätze der anderen wenigstens einen Röntgeneinrichtung anzuzeigen, so dass insbesondere auch bei geschickter Platzierung der Röntgeneinrichtungen beispielsweise von beiden Seiten eines Patiententisches zur Lagerung des Patienten aufgenommene Bilddatensätze aller Röntgeneinrichtungen betrachtet werden können, was die Ergonomie der vorgeschlagenen Ausgestaltungen weiter erhöht.

Ein anderer wesentlicher Vorteil der Ausgestaltung ist, dass die mobile Röntgeneinrichtung zum einen dank der eigenen Steuereinrichtung auch unabhängig von der restlichen wenigstens einen Röntgeneinrichtung eingesetzt werden kann, zum anderen aber auch bezüglich der Röntgenanordnung optional ist, mithin bei Bedarf hinzugefügt werden kann und dann, wenn sie nicht benötigt wird, platzsparend entfernt werden kann. Das bedeutet, beispielsweise ist eine Ausgestaltung mit zwei Röntgeneinrichtungen denkbar, die zum einen getrennt an zwei Patiententischen/in zwei Räumen eingesetzt werden können, aber in seltener auftretender Fällen, in denen eine Mehr-Ebenen-Bildgebung erforderlich ist, miteinander gekoppelt werden können, indem die Röntgeneinrichtungen gemeinsam an einem Patiententisch vereint werden und die wenigstens eine Kommunikationsverbindung aufgebaut wird. Auf diese Weise ist eine äußerst flexible und kosteneffektive Lösung gegeben.

Wird eine ebenso mobile Zwischeneinrichtung, beispielsweise ein Monitorwagen, für mehrere insbesondere mobile Röntgeneinrichtungen genutzt, besteht mithin die Möglichkeit, an eine derartige Zwischeneinrichtung, insbesondere einen Monitorwagen anstatt nur einer Röntgeneinrichtung zwei oder mehr Röntgeneinrichtungen anzuschließen. Es wird nur noch eine solche Zwischeneinrichtung im Raum benötigt; zudem benötigt ein Benutzer insbesondere nur noch ein einziges Bedienelement, wie beispielsweise einen Fußschalter. Eine Platzersparnis führt grundsätzlich zu einem flüssigeren Ablauf im Untersuchungs- und/oder Behandlungsraum und spart somit Zeit ein. Die fehlende Zwischeneinrichtung, insbesondere der fehlende Monitorwagen, vermindert das Risiko, über Kabel zu stürzen. Da nur noch eine einzige Darstellungseinrichtung im Fall eines Monitorwagens notwendig ist, muss ein Benutzer nicht überlegen, wo sein gerade aufgenommener Röntgenbilddatensatz angezeigt wird. Dies ermöglicht ein konzentriertes Arbeiten. Soll, insbesondere in einem solchen Fall, eine drahtgebundene Kommunikationsverbindung verwendet werden, können als Kommunikationsschnittstelle wenigstens zwei entsprechende Anschlüsse an der Zwischeneinrichtung, insbesondere den Monitorwagen vorgesehen werden, beispielsweise zwei Anschlussbuchsen.

Zur Erhöhung der Sicherheit kann vorgesehen sein, dass wenigstens eine der Steuereinrichtungen bei dem Aufbau der Kommunikationsverbindung zur Authentifizierung des wenigstens einen Kommunikationspartners, insbesondere unter Einbindung eines Benutzers, ausgebildet ist. Auf diese Weise können Fehlnutzungen sowie unbefugte Nutzungen bzw. eine Kopplung zwischen nicht zur Kopplung vorgesehenen Röntgeneinrichtungen vermieden werden. Beispielsweise kann eine Authentifizierung die benutzerseitige Eingabe einer PIN umfassen. Selbstverständlich können zusätzlich oder alternativ auch andere, im Stand der Technik grundsätzlich bekannte Authentifizierungsverfahren eingesetzt werden.

In einer besonders vorteilhaften Ausgestaltung der vorliegenden Erfindung kann vorgesehen sein, dass eine als Master ausgebildete oder benutzerseitig und/oder automatisch als Master bestimmte Steuereinrichtung zur koordinierten Steuerung der gesamten Röntgenanordnung ausgebildet ist. Das bedeutet, wenn sich nicht, beispielsweise in Form einer Steuereinrichtung einer Zwischeneinrichtung, eine ohnehin zentrale Steuerungsinstanz anbietet, kann eine Steuereinrichtung als eine Master-Steuereinrichtung bestimmt werden, um eine zentrale, koordinierte Steuerung der Röntgenanlage umzusetzen. Die übrigen Steuereinrichtungen können dann als Slave-Steuereinrichtungen aufgefasst werden. Ist eine fest installierte Röntgeneinrichtung unter den Röntgeneinrichtungen, so wird deren Steuereinrichtung bevorzugt automatisch bzw. festgelegt als Master-Steuereinrichtung verwendet. Die Master-Steuereinrichtung ist dabei, wie grundsätzlich bekannt, den Slave-Steuereinrichtungen hierarchisch übergeordnet und stellt insbesondere bevorzugt Steuerdaten für den Bildaufnahmebetrieb aller Röntgeneinrichtungen und/oder die Bildverarbeitung für Bilddatensätze aller Röntgeneinrichtungen bereit. In Fällen, in denen sich keine Steuereinrichtung besonders als Master-Steuereinrichtung anbietet, beispielsweise, wenn zwei gleichberechtigte mobile Röntgeneinrichtungen vorhanden sind, kann die Rolle der Master-Steuereinrichtung seitens eines Benutzers und/oder automatisch, beispielsweise nach dem Zufallsprinzip, vergeben werden. Dabei ist eine benutzerseitige Auswahl bevorzugt, nachdem dann eine der Röntgeneinrichtungen ausgewählt werden kann, die hinsichtlich der Bedienung besser positioniert ist.

Wird eine Master-Steuereinrichtung verwendet, kann zweckmäßigerweise, falls die Master-Steuereinrichtung einer der Röntgeneinrichtungen zugeordnet ist oder dieser zugehörig ist, und diese Röntgeneinrichtung eine Bedieneinrichtung und eine Darstellungseinrichtung aufweist, vorgesehen sein, dass Bedieneinrichtungen und/oder Darstellungseinrichtungen anderer Röntgeneinrichtungen zumindest zeitweise deaktiviert werden. Das bedeutet, auch von der benutzerseitigen Bedienung kann vorgesehen sein, die Röntgeneinrichtung, der die Master-Steuereinrichtung zugehörig oder zugeordnet ist, besonders hervorzuheben, so dass eine Bedienungsverwirrung oder dergleichen bereits im Voraus möglichst weitgehend vermieden wird.

Die koordinierte Steuerung von Röntgeneinrichtungen kann insbesondere auch die übergreifende, aufeinander abgestimmte Auswahl wenigstens eines Aufnahmeparameters für jeweilig in ihrem Aufnahmebetrieb zu koordinierende Röntgeneinrichtungen umfassen. So kann die als Master ausgebildete Steuereinrichtung beispielsweise zur Auswahl unterschiedlicher Röntgenspektren für Röntgeneinrichtungen, deren Bilddatensätze gemeinsam ausgewertet werden sollen, ausgebildet sein. Auf diese Weise lässt sich beispielsweise elegant eine Mehrenergie-Bildgebung, insbesondere eine Dualenergie-Bildgebung, realisieren. Ein anderes Beispiel für eine Zuordnung unterschiedlicher Aufnahmeparameter bei einem gemeinsamen Bildaufnahmebetrieb ist die Zuordnung unterschiedlicher abzudeckender Bereiche im Raum zu verwendender Aufnahmegeometrien zu Röntgeneinrichtungen. Beispielsweise kann bei der Verwendung von zwei C-Bögen jeder dieser C-Bögen einen Anteil zu verwendender Projektionswinkel überstreichen.

Es sei an dieser Stelle noch angemerkt, dass eine Master-Steuereinrichtung für einen derartigen koordinierten Betrieb nicht zwangsläufig erforderlich ist, wenn ein entsprechendes Verhandlungsverfahren zwischen Steuereinrichtungen unterschiedlicher Röntgeneinrichtungen umgesetzt wird.

In einer besonders bevorzugten Ausgestaltung der vorliegenden Erfindung kann vorgesehen sein, dass den Aufnahmeanordnungen mittels der jeweiligen Steuereinrichtungen ansteuerbare Verstelleinrichtungen zur Einstellung unterschiedlicher Aufnahmegeometrien zugeordnet sind, wobei die als Master dienende Steuereinrichtung zur bewegungskoppelnden Ansteuerung der Verstelleinrichtungen von wenigstens zwei der wenigstens zwei Röntgeneinrichtungen ausgebildet ist. Das bedeutet konkret, dass die Verstelleinrichtungen einer Röntgeneinrichtung, der die Master-Steuereinrichtung nicht zugehörig ist, durch Übermittlung von Steuerdaten über die Kommunikationsverbindung an die der Röntgeneinrichtung, der die Verstelleinrichtungen zugehörig sind, zugeordnete Steuereinrichtung erfolgt, welche die Steuerdaten zur Ansteuerung der Verstelleinrichtungen umsetzt. Auf diese Weise wird im Rahmen der vorliegenden Erfindung eine Bewegungskopplung der Aufnahmeanordnungen aneinander erreicht, was besonders bevorzugt bei der Verwendung von C-Bögen aufweisenden Röntgeneinrichtungen einsetzbar ist. So kann beispielsweise vorgesehen sein, dass die als Master dienende Steuereinrichtung zur Aufrechterhaltung wenigstens eines konstanten Winkels zwischen den Ausrichtungen der gekoppelt verstellten Aufnahmeanordnungen ausgebildet ist.

Dabei sei nochmals hervorgehoben, dass eine Voraussetzung für einen derart gekoppelten Betrieb Verstelleinrichtungen sind, die eine zumindest teilweise automatisierte Einstellung von Aufnahmegeometrien ermöglichen. In einer konkreten Ausgestaltung können beispielsweise zwei Röntgeneinrichtungen mit jeweils einem die Aufnahmeanordnung tragenden C-Bogen verwendet werden, wobei zunächst die C-Bögen drehbar in einer gemeinsamen Ebene angeordnet werden, aber mit einem vorbestimmten Winkelversatz gegeneinander, so dass entsprechende Bilder auch mit einem entsprechenden Unterschied der Projektionswinkel als konkret unterschiedliche Aufnahmegeometrien aufgenommen werden. Diese Einstellung eines relativen Winkels kann, insbesondere wenn eine Art von Absoluteinstellung mit einer klaren Referenz abgerufen werden kann und/oder eine anderweitige Registrierung besteht, auch automatisch eingestellt werden. In jedem Fall ist es zweckmäßig, wenn über die Kommunikationsverbindung wenigstens die aktuellen Winkelstellungen der C-Bögen ausgetauscht werden können. Weisen nun beispielsweise die Aufnahmeanordnungen bei der Bewegungskopplung der Röntgeneinrichtungen und/oder nach einer gezielten Einstellung unmittelbar nach der Kopplung einen bestimmten Winkelunterschied, beispielsweise 90°, auf, wird dieser Unterschied konstant gehalten, egal wie stark einer der C-Bögen gedreht wird. Wird beispielsweise der C-Bogen einer ersten der Röntgeneinrichtungen um 25° gedreht, wird der C-Bogen der anderen Röntgeneinrichtung gleichzeitig automatisch ebenso um 25° gedreht.

Diese Winkelkopplung (oder im Allgemeinen Bewegungskopplung) kann zweckmäßigerweise bei um unterschiedliche Achsen mittels der Verstelleinrichtungen drehbaren C-Bögen für alle diese möglichen Drehachsen gelten. So ist es beispielsweise denkbar, dass eine Winkelkopplung sowohl für eine LAO/RAO-Drehung als auch für eine CRA/CAU-Drehung gelten kann. Auf diese Weise wird eine sphärisch synchronisierte Bewegung gekoppelter C-Bögen möglich, von denen wenigstens einer einer mobilen Röntgeneinrichtung zugehörig ist. Eine Bewegungskopplung über die Konstanthaltung von Differenzwinkeln der unterschiedlichen Aufnahmeanordnungen zueinander ist der häufigste Anwendungsfall der vorliegenden Erfindung in diesem Ausführungsbeispiel Allgemein kann jedoch hinsichtlich des Beispiels von zwei unterschiedlichen Röntgeneinrichtungen zugehörigen C-Bögen und deren Kopplung gesagt werden, dass auf einfache Weise aus grundsätzlich unabhängigen Röntgeneinrichtungen eine Funktionalität geschaffen werden kann, wie sie bei einer fest installierten Biplan-Röntgeneinrichtung gegeben ist, wobei jedoch eine große Flexibilität dadurch gewonnen wird, dass die mobile Röntgeneinrichtung nur vor Ort sein muss, wenn sie dort auch tatsächlich benötigt wird.

Dabei sei an dieser Stelle noch angemerkt, dass es in einer alternativen Ausgestaltung, in der nur eine der Röntgeneinrichtungen über die notwendigen Verstelleinrichtungen verfügt, auch denkbar ist, dass für wenigstens zwei gekoppelt zu verstellende Aufnahmeanordnungen einer der Aufnahmeanordnungen eine Messeinrichtung zur Vermessung einer manuellen Verstellung der Aufnahmeanordnung zugeordnet ist und den übrigen Aufnahmeanordnungen mittels der jeweiligen Steuereinrichtung ansteuerbare Verstelleinrichtungen zur Einstellung unterschiedlicher Aufnahmegeometrien zugeordnet sind, wobei die als Master dienende Steuereinrichtung zur bewegungskoppelnden Ansteuerung der Verstelleinrichtungen von wenigstens einer weiteren der wenigstens zwei Röntgeneinrichtungen in Abhängigkeit einer Messung der Messeinrichtung ausgebildet ist. Weist also eine der Röntgeneinrichtungen keine automatischen Möglichkeiten zur Einstellung einer Aufnahmegeometrie auf, können dort Messeinrichtungen vorgesehen werden, die die aktuelle Stellung nachverfolgen und deren Messwerte herangezogen werden können, um die Bewegungskopplung an den anderen Röntgeneinrichtungen, die entsprechende Verstelleinrichtungen aufweisen, zu realisieren.

Wie bereits erwähnt, kann es, insbesondere im Hinblick auf eine gekoppelte Verwendung von Aufnahmegeometrien, ausreichend sein, wenn entsprechende Mittel zur Erfassung aktueller Einstellungen der Aufnahmeanordnung an den einzelnen Röntgeneinrichtungen vorliegen und ein klarer Bezugspunkt definiert, so dass durch Nutzung der Kommunikationsverbindung relative Positionsinformationen für die Röntgeneinrichtungen hinreichend genau vorliegen. Wird beispielsweise bei C-Bögen davon ausgegangen, dass die C-Bögen bereits manuell auf eine gemeinsame Drehebene eingestellt sind, kann ein einfacher Austausch von aktuellen Winkelstellungen hinsichtlich eines klar definierten Bezugspunkts ausreichend sein, um festzustellen, wie der relative Winkel zwischen den C-Bögen aktuell ist. Für einige Anwendungen kann es jedoch zweckmäßiger sein, wenn eine tatsächliche, insbesondere vollständige, Registrierung zwischen Koordinatensystemen der unterschiedlichen Röntgeneinrichtungen vorliegt.

So kann vorgesehen sein, dass wenigstens eine Steuereinrichtung, insbesondere wenigstens die als Master ausgebildete Steuereinrichtung, eine Registrierungseinheit zur Registrierung von Koordinatensystemen der unterschiedlichen Röntgeneinrichtungen aufweist. Dies ermöglicht es insbesondere, dass die Position von Komponenten einer Röntgeneinrichtung auch in Steuereinrichtungen der anderen Röntgeneinrichtungen bekannt gemacht werden kann.

In diesem Kontext sieht eine besonders vorteilhafte Ausgestaltung vor, dass wenigstens eine einer Röntgeneinrichtung zumindest zugeordnete Steuereinrichtung eine Kollisionsvermeidungseinheit für diese Röntgeneinrichtung aufweist, wobei die Kollisionsvermeidungseinheit zur Berücksichtigung von über die Kommunikationsverbindung erhaltenen Positionsinformationen wenigstens einer weiteren Röntgeneinrichtung ausgebildet ist. Mit besonderem Vorteil kann dabei die wenigstens eine mobile Röntgeneinrichtung bei der Kollisionsvermeidung der wenigstens einen fest installierten Röntgeneinrichtung berücksichtigt werden. Auf diese Weise wird die Tiefe der Integration zu einem gemeinsamen Röntgengerät, wie sie eingangs angesprochen wurde, erhöht, nachdem insbesondere eine die gesamte Röntgenanordnung umfassende Kollisionsvermeidungsstrategie in der Kollisionsvermeidungseinheit umgesetzt werden kann, um die Sicherheit des Patienten und die Beschädigungsfreiheit von Komponenten möglichst weitgehend zu gewährleisten. Hier ist eine Registrierung besonders nützlich, um die Positionen der Komponenten anderer Röntgeneinrichtungen möglichst genau kennen zu können.

Konkret kann vorgesehen sein, dass die Registrierungseinheit zur Registrierung der Koordinatensysteme anhand von an den Röntgeneinrichtungen und/oder in dem von den Röntgeneinrichtungen genutzten Raum vorgesehenen Markern ausgebildet ist. Dabei sind verschiedene konkrete, Marker nutzende Ansätze denkbar, um die Registrierung zwischen Koordinatensystemen herzustellen. So können beispielsweise bodenseitige Marker in dem genutzten Raum vorgesehen sein, auf die die wenigstens eine mobile Röntgeneinrichtung zu Beginn der Kopplung positioniert werden kann, um eine definierte Initialposition herzustellen, auf die sich später eintretende Bewegungen beziehen. Derartige Bodenmarkierungen können auch zur Repositionierung der mobilen Röntgeneinrichtung oder dergleichen dienen. Mit besonderem Vorteil kann in diesem Kontext auch eine Detektionseinrichtung zur Erfassung der Marker, insbesondere eine optische und/oder elektromagnetische Detektionseinrichtung, vorgesehen sein. Das bedeutet, beispielsweise Bodenmarkierungen können elektromagnetisch und/oder optisch erkannt werden. Jedoch auch andere Arten von Markern, beispielsweise bekannte Merkmale des Patiententisches oder dergleichen, können mit beispielsweise an der mobilen Röntgeneinrichtung, insbesondere am Träger, vorgesehenen Detektionseinrichtungen erfasst werden, so dass die Position der mobilen Röntgeneinrichtung im Raum möglichst genau erfasst werden kann. Selbstverständlich können auch andere Arten von im Stand der Technik bereits grundsätzlich vorgeschlagenen Positionsbestimmungssystemen eingesetzt werden, beispielsweise solche, die elektromagnetische Marker oder dergleichen nutzen.

Eine besonders zweckmäßige Ausgestaltung der vorliegenden Erfindung sieht vor, dass wenigstens eine Steuereinrichtung, insbesondere die als Master ausgebildete Steuereinrichtung, zur Ermittlung von eine vorbestimmte relative Positionierung wenigstens der Aufnahmeanordnungen von wenigstens zwei der wenigstens zwei Röntgeneinrichtungen herstellenden Anweisungsdaten ausgebildet ist, welche zur manuellen Einstellung über eine Darstellungseinrichtung ausgebbar und/oder wenigstens teilweise zur Ansteuerung von der Positionsanpassung dienenden Komponenten wenigstens einer Röntgeneinrichtung verwendet werden, insbesondere nach einer Übertragung über wenigstens eine der wenigstens einen Kommunikationsverbindung. Insbesondere also dann, wenn beispielsweise Mobilitätseinrichtungen mobiler Röntgeneinrichtungen auch Antriebsvorrichtungen aufweisen, können diese eingesetzt werden, um automatisch geeignete Relativpositionen zwischen den Röntgeneinrichtungen herzustellen, die sich auf bestimmte gewünschte Relativpositionen der Aufnahmeanordnungen beziehen, welche dann beispielsweise über entsprechende, bereits erwähnte Verstelleinrichtungen herbeigeführt werden können. Möglich ist es aber auch, geeignete Relativpositionierungen der Röntgeneinrichtungen, insbesondere der wenigstens einen mobilen Röntgeneinrichtung, herbeizuführen, in dem entsprechende Anweisungen an einen Benutzer ausgegeben werden, die sich beispielsweise auf die bereits erwähnten Bodenmarkierungen beziehen können. So kann auch manuell eine bestimmte Positionierung hergestellt werden.

In Weiterbildung der vorliegenden Erfindung kann vorgesehen sein, dass wenigstens eine der Steuereinrichtungen eine Synchronisationseinheit zur Synchronisierung von Zeitgebern von wenigstens zwei über wenigstens eine der wenigstens einen Kommunikationsverbindung verbundenen Röntgeneinrichtungen aufweist. Insbesondere können die beiden zu synchronisierenden Steuereinrichtungen jeweils eine Synchronisierungseinheit aufweist. Die Synchronisierung kann seitens der Steuereinrichtungen der Röntgeneinrichtungen genutzt werden, um gleiche Pulsraten und/oder gleiche Aufnahmezeitpunkte und/oder dergleichen zu erreichen. Verschiedene Varianten zur konkreten Synchronisierung von Röntgeneinrichtungen sind einsetzbar, beispielsweise eine schnelle Software-Synchronisation und/oder die Verwendung dedizierter Hardware-Leitungen.

Eine zweckmäßige Weiterbildung der Erfindung sieht ferner vor, dass die Röntgenanordnung wenigstens einen fest installierten Generator und/oder wenigstens eine fest installierte Kühleinrichtung mit Anschlüssen für die wenigstens eine mobile Röntgeneinrichtung aufweist. Für eine Leistungssteigerung der mobilen Röntgeneinrichtung kann diese mithin im Stationärbetrieb an einen eigenen, fest installierten Generator und/oder eine zusätzliche Kühleinrichtung, die ebenso fest installiert ist, angeschlossen werden.

Vorzugsweise kann wenigstens eine der Röntgeneinrichtungen, insbesondere eine fest installierte Röntgeneinrichtung, ein Bildsystem aufweisen, wobei wenigstens eine weitere der Röntgeneinrichtungen, insbesondere eine mobile Röntgeneinrichtung, zur Mitnutzung des Bildsystems ausgebildet ist, insbesondere durch Übertragung von Bilddaten über die Kommunikationsverbindung. Das bedeutet, in besonders vorteilhaften Ausgestaltungen der vorliegenden Erfindung kann wenigstens eine der wenigstens einen mobilen Röntgeneinrichtung Bilddaten über wenigstens eine der wenigstens einen Kommunikationsverbindung an eine weitere, insbesondere eine fest installierte, Röntgeneinrichtung mit einem Bildsystem übertragen und dieses Bildsystem sozusagen mitnutzen, insbesondere im Hinblick auf eine gemeinsame Auswertung der Bilddaten. Das bedeutet, das Bildsystem kann mit besonderem Vorteil zur gemeinsamen Auswertung von Bilddaten mehrerer Röntgeneinrichtungen ausgebildet sein. Beispielsweise kann also eine Rekonstruktion eines dreidimensionalen Bilddatensatzes aus Projektionsbilder-Bilddatensätzen mehrerer Röntgeneinrichtungen durch das Bildsystem erfolgen. Dabei kann die das Bildsystem mitnutzende Röntgeneinrichtung sowohl fertig vorverarbeitete Bilddatensätze, beispielsweise im DICOM-Format, an das Bildsystem der anderen Röntgeneinrichtung übergeben als auch Roh-Bilddaten, die dann in dem Bildsystem in der jeweiligen Bildkette verarbeitet werden. 3D-Akquisitionsläufe können mit derart gekoppelten Röntgeneinrichtungen einzeln durchgeführt werden, jedoch ist es auch möglich, einen synchronisierten Lauf mit beiden Röntgeneinrichtungen vorzunehmen, in diesem Fall auch mit unterschiedlichen Aufnahmeparametern, um unterschiedliche Röntgenspektren abzudecken, wie dies eingangs bereits erläutert wurde.

Insgesamt kann die Röntgenanordnung wenigstens eine Darstellungseinrichtung für mit den Röntgeneinrichtungen aufgenommene Bilddatensätze aufweisen. Dabei können beispielsweise auch die Röntgeneinrichtungen selber Darstellungseinrichtungen aufweisen und/oder eine Zwischeneinrichtung, beispielsweise ein Monitorwagen, mit einer Darstellungseinrichtung kann verwendet werden. Dabei kann es sowohl sinnvoll sein, bis auf eine Darstellungseinrichtung alle anderen Darstellungseinrichtungen zu deaktivieren und Bilddatensätze aller Röntgeneinrichtungen nur auf dieser einen Darstellungseinrichtung darzustellen, als auch in manchen Umgebungen und/oder für bestimmte Untersuchungs- und/oder Behandlungsaufgaben zweckmäßigerweise die Möglichkeit zur Darstellung von Bilddatensätzen aller Röntgeneinrichtungen auf mehreren oder sogar allen vorhandenen Darstellungseinrichtungen zu bieten. Dies ist aufgrund der wenigstens einen Kommunikationsverbindung im Rahmen der vorliegenden Erfindung problemlos möglich, da die entsprechenden Bilddatensätze zwischen den Röntgeneinrichtungen bzw. der Zwischeneinrichtung, mithin den Orten der unterschiedlichen Darstellungseinrichtungen, ausgetauscht werden können. Bei den Darstellungseinrichtungen kann es sich insbesondere um wenigstens einen Monitor handeln.

Eine besonders vorteilhafte Ausgestaltung in diesem Zusammenhang sieht vor, dass die Darstellungseinrichtung zur Darstellung von unter unterschiedlichen Projektionswinkeln, insbesondere mit unterschiedlichen Röntgeneinrichtungen, aufgenommenen zweidimensionalen Projektionsbildern als Bilddatensätze gemeinsam in einer 3D-Visualisierung nach Art eines Buches ausgebildet ist, wobei jede dargestellte Seite des Buches einem Projektionsbild entspricht und die Seiten gegeneinander in einem Winkel, der dem Projektionswinkelunterschied der jeweiligen Projektionsbilder entspricht, aufgestellt wird. Auf diese Weise kann ein Projektionswinkelunterschied zwischen den Bildebenen unmittelbar in einem buchartigen Stil dargestellt werden, nachdem der Projektionswinkelunterschied deutlich durch den Winkelunterschied der Seiten des Buches dargelegt wird. Wenn mehr als zwei Projektionsbilder auf diese Art und Weise angezeigt werden sollen, können auch zusätzliche Seiten zu dem "Buch" hinzugefügt werden. Dies führt zu einer äußerst intuitiven, chirurgischen Darstellung von zweidimensionalen Bilddatensätzen. Der Benutzer kann mit ergonomischem Einblick in die Projektionswinkelunterschiede durch das Buch blättern, wobei dieses Darstellungsverfahren auch unabhängig von der hier beschriebenen Kopplung von Röntgeneinrichtungen über eine Kommunikationsverbindung zweckmäßig eingesetzt werden kann.

So ist grundsätzlich ein Verfahren zur Darstellung von zweidimensionalen, mit wenigstens einer medizinischen Bildaufnahmeeinrichtung aufgenommenen Bildern auf einer Darstellungseinrichtung denkbar, welche sich dadurch auszeichnet, dass die Bilder gemeinsam in einer 3D-Visualisierung nach Art eines Buches dargestellt werden, wobei jede dargestellte Seite des Buches einem Bild entspricht und die Seiten gegeneinander in einem Winkel, der dem Winkel der Bildebenen der jeweiligen Bilder entspricht, aufgestellt sind. Beispielsweise kann ein derartiges Visualisierungsverfahren bei Bildgebungsmodalitäten wie der Computertomographie und/oder der Magnetresonanzbildgebung eingesetzt werden und zeigt auch dort auf intuitive Weise neben den Bildern auch unmittelbar deren Winkelverhältnisse zueinander an, indem die Seiten des Buches in dem entsprechenden Winkelunterschied zueinander stehend dargestellt werden. Auch im allgemeinen Fall können weitere zweidimensionale Bilder durch Hinzufügen weiterer Buchseiten einfach ergänzt werden. Über entsprechende Bedienelemente, die über eine entsprechende Bedieneinrichtung bzw. Benutzeroberfläche bereitgestellt werden können, kann es ermöglicht werden, durch das so dargestellte Buch zu blättern. So ist ein intuitiverer Zugang zu Mehr-Ebenen-Bilddaten gegeben, da bei der bisherigen Darstellung der Bilder Seite-an-Seite der räumliche/winkelbezogene Zusammenhang aus anderen Informationen schlussgefolgert werden musste, beispielsweise aus einer für den Benutzer sichtbaren relativen Stellung von C-Bögen zueinander.

In einer konkreten Ausgestaltung der vorliegenden Erfindung kann vorgesehen sein, dass eine der Röntgeneinrichtungen eine fest installierte, deckengehängte Röntgeneinrichtung mit einem C-Bogen, der die Aufnahmeanordnung trägt, oder eine Sliding Gantry-CT-Einrichtung ist, wobei deren Bedien- und/oder Darstellungseinrichtungen aufgrund der wenigstens einen Kommunikationsverbindung auch zur Nutzung mit der wenigstens einen mobilen Röntgeneinrichtung ausgebildet sind. Beispielsweise kann also ein sogenannter mobiler C-Bogen als zweite Ebene bei einem deckengehängten Angiographie-System, also einer fest installierten Röntgeneinrichtung mit einem C-Bogen, verwendet werden, um Biplan-Funktionen bereitzustellen. Zusätzlich existiert hier jedoch der Vorteil eines besseren Zugangs zum Patienten bzw. Patiententisch, da die mobile Röntgeneinrichtung weit entfernt "geparkt" werden kann. Ein weiterer Vorteil ist, dass beide Röntgeneinrichtungen auch einzeln verwendbar sind, wie auch allgemein bereits dargelegt wurde.

Dabei kann mit besonderem Vorteil eine Andockbarkeit mit tiefer Integration, also insbesondere Inklusion der mobilen Röntgeneinrichtung auch bezüglich der Bedieneinrichtungen, der Darstellungseinrichtungen, der Kollisionsvermeidungseinheiten, der Strahlungskette und der Bildkette, vorgesehen werden, um die mobile Röntgeneinrichtung als Bestandteil eines klar definierten Biplan-Röntgengeräts etablieren, wobei gleichzeitig die Vorteile beider einzelnen Röntgeneinrichtungen beibehalten werden, insbesondere hinsichtlich Mobilität bzw. Genauigkeit. Der Zugang zum Patienten wird verbessert und Kombinationen aus Biplan-Angiographieeinrichtungen mit Sliding Gantry-CT-Einrichtungen sind auch ohne die eingangs beschriebene Patientendrehung möglich, wenn der Träger der mobilen Röntgeneinrichtung frei bewegt werden kann und aus dem Bewegungspfad der Sliding Gantry-CT-Einrichtung entfernt werden kann.

Dabei ist an dieser Stelle noch anzumerken, dass sich selbstverständlich alle bezüglich der erfindungsgemäßen Röntgenanordnung beschriebenen Merkmale und Vorteile entsprechend auf das erfindungsgemäße Verfahren übertragen lassen. Insbesondere lassen sich beschriebene Ausbildungen der Steuereinrichtung, des Bildsystems und/oder von Subeinheiten auch als im Rahmen des erfindungsgemäßen Verfahrens durchgeführte Schritte verstehen.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Prinzipskizze einer ersten Ausgestaltung einer erfindungsgemäßen Röntgenanordnung,
- Fig. 2: eine Prinzipskizze einer zweiten Ausgestaltung einer erfindungsgemäßen Röntgenanordnung,
- Fig. 3: eine Prinzipskizze einer dritten Ausgestaltung einer erfindungsgemäßen Röntgenanordnung,
- Fig. 4: mögliche Komponenten von Steuereinrichtungen der erfindungsgemäßen Röntgenanordnungen,
- Fig. 5: eine erste konkrete Ausgestaltung einer erfindungsgemäßen Röntgenanordnung,
- Fig. 6: eine zu Fig. 5 alternative Darstellung nach Art eines Schichtprinzips,
- Fig. 7: einen Ablaufplan eines Ausführungsbeispiels des erfindungsgemäßen Verfahrens,
- Fig. 8: eine Möglichkeit zur Darstellung zweidimensionaler Bilddatensätze mit unterschiedlichen Aufnahmegeometrien,
- Fig. 9: eine zweite konkrete Ausführungsform einer erfindungsgemäßen Röntgenanordnung, und
- Fig. 10: eine dritte konkrete Ausführungsform einer erfindungsgemäßen Röntgenanordnung.

Die im Folgenden dargestellten Ausführungsbeispiele der vorliegenden Erfindung sind jeweils für zwei kommunikationstechnisch verbindbare Röntgeneinrichtungen einer Röntgenanordnung gezeigt; die hier dargestellten Prinzipien lassen sich jedoch auch auf Röntgenanordnungen mit mehr als zwei Röntgeneinrichtungen problemlos übertragen.

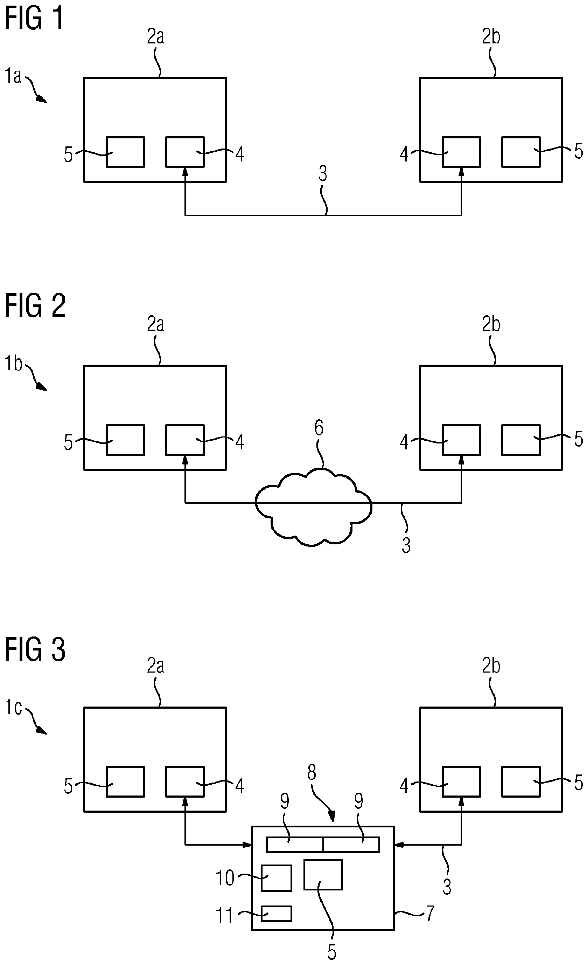

Die Fig. 1 bis 3 zeigen grundsätzliche Möglichkeiten zur Realisierung der vorliegenden Erfindung. In der in Fig. 1 gezeigten Röntgenanordnung 1a sind zwei Röntgeneinrichtungen 2a, 2b über eine Kommunikationsverbindung 3 direkt und unmittelbar verbindbar. Zum Aufbau der Kommunikationsverbindung 3 weisen die Röntgeneinrichtungen 2a, 2b, von denen wenigstens eine mobil ausgestaltet ist, Kommunikationsschnittstellen 4 auf, die über jeweilige Steuereinrichtungen 5 der Röntgeneinrichtungen 2a, 2b ansprechbar sind. Ist eine drahtgebundene Kommunikationsverbindung 3 vorgesehen, können die Kommunikationsschnittstellen entsprechende Anschlüsse aufweisen; handelt es sich um eine rein drahtlose Kommunikationsverbindung 3, beispielsweise über Bluetooth und/oder WLAN, sind die Kommunikationsschnittstellen 4 als Funkschnittstellen ausgebildet.

Fig. 2 zeigt eine modifizierte zweite Ausgestaltung einer Röntgenanordnung 1b, die sich von der Ausgestaltung der Fig. 1 darin unterscheidet, dass die Kommunikationsverbindung 3 nun über ein drahtloses Netzwerk 6 hergestellt wird, das am Einsatzort der Röntgeneinrichtungen 2a, 2b ohnehin vorliegt. Das bedeutet, die Kommunikationsverbindung 3 kann indirekt über eine Netzwerkkomponente des Netzwerks 6 hergestellt sein. Eine solche Netzwerkkomponente kann beispielsweise ein Krankenhausinformationssystem (HIS) und/oder ein Radiologieinformationssystem (RIS) sein.

In der dritten grundsätzlichen Ausgestaltung der Fig. 3 weist die Röntgenanordnung 1c zusätzlich zu den Röntgeneinrichtungen 2a, 2b eine Zwischeneinrichtung 7, beispielsweise in Form eines Monitorwagens, auf, die ebenso eine Steuereinrichtung 5 besitzt, die eine Kommunikationsschnittstelle 8 der Zwischeneinrichtung 7 ansprechen kann. Die Kommunikationsschnittstelle 8 weist dabei mehrere, je nach Ausgestaltung der Kommunikationsverbindungen 3, drahtgebundene und/oder drahtlose Anschlüsse 9 auf, so dass Kommunikationsverbindungen 3 zu allen Röntgeneinrichtungen 2a, 2b aufgebaut werden können. Zweckmäßigerweise weist die Zwischeneinrichtung 7 vorliegend auch eine Darstellungseinrichtung 10, insbesondere einen Monitor, und eine Bedieneinrichtung 11, auf, was die Bedienung beider Röntgeneinrichtungen 2a, 2b von der Zwischeneinrichtung 7 aus genauso erlaubt wie das Anzeigen von Bilddatensätzen beider Röntgeneinrichtungen 2a, 2b auf der Darstellungseinrichtung 10.

In den hier dargestellten Ausführungsbeispielen wird jeweils eine der vorhandenen Röntgeneinrichtungen 5 als eine Master-Steuereinrichtung verwendet, wobei die anderen Steuereinrichtungen 5 als Slaves verstanden werden können. Das bedeutet, eine der Steuereinrichtungen 5, nämlich die als Master festgelegte, bestimmte bzw. ausgewählte Steuereinrichtung 5, wird hierarchisch höher angesiedelt als die übrigen, als Slave funktionierenden Steuereinrichtungen 5, wobei bevorzugt die als Master bestimmte Steuereinrichtung 5 zentral koordiniert den Betrieb aller Röntgeneinrichtungen 2a, 2b der jeweiligen Röntgenanordnung 1a, 1b, 1c steuert. Hierfür kann die als Master bestimmte Steuereinrichtung 5 Steuerdaten an als Slaves bestimmte Steuereinrichtungen 5 übermitteln, welche diese zum Steuern des Aufnahmebetriebs der jeweiligen Röntgeneinrichtung 2a, 2b nutzen können; gleichzeitig kann die als Master bestimmte Steuereinrichtung 5 auch Bilddatensätze von insbesondere anderen Röntgeneinrichtungen 2a, 2b empfangen und zur gemeinsamen Weiterverarbeitung, insbesondere auch Darstellung, nutzen. Dabei ist es insbesondere die wenigstens eine mobile Röntgeneinrichtung 2a und/oder 2b, deren Steuereinrichtung 5 als Slave-Steuereinrichtung verwendet wird, so dass die entsprechende mobile Röntgeneinrichtung 2a und/oder 2b letztlich fernsteuerbar angekoppelt wird.

Im Fall der Röntgenanordnung 1c wird zweckmäßigerweise die Steuereinrichtung 5 der Zwischeneinrichtung 7 als Master-Steuereinrichtung verwendet; in dem Fall, in dem eine der Röntgeneinrichtungen 2a, 2b eine fest installierte Röntgeneinrichtung 2a, 2b ist, kann bevorzugt auch die Steuereinrichtung 5 dieser fest installierten Röntgeneinrichtung 2a oder 2b als Master-Steuereinrichtung herangezogen werden, insbesondere festgelegt sein. Im Fall zweier mobiler Röntgeneinrichtungen 2a, 2b, falls keine Zwischeneinrichtung 7 vorhanden ist, kann benutzerseitig auswählbar sein, welche Steuereinrichtung 5 die Master-Steuereinrichtung ist; auch eine automatische, beispielsweise auf der Leistungsfähigkeit basierende und/oder zufallsbeeinflusste Wahl ist denkbar.

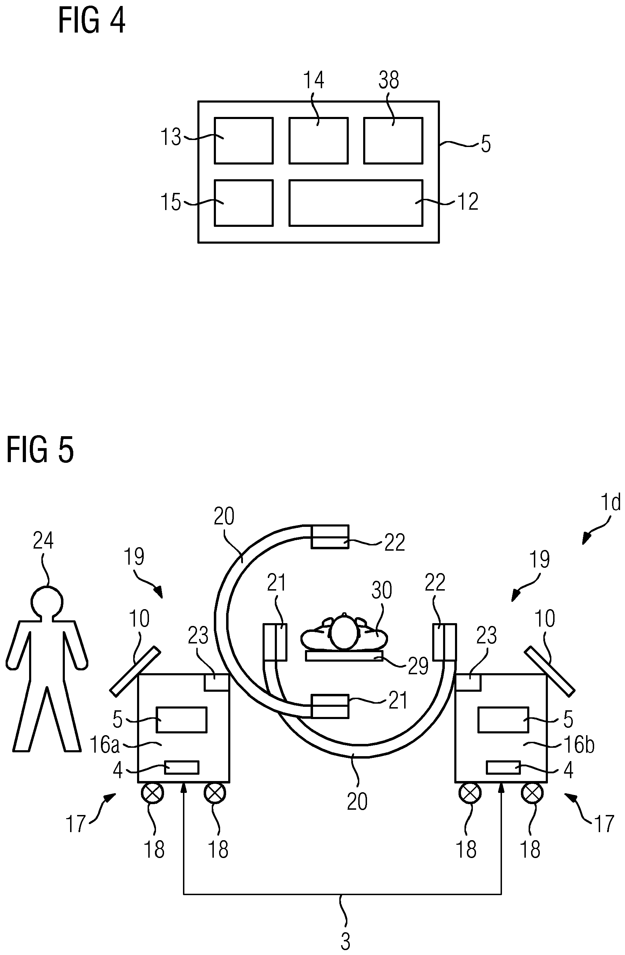

Fig. 4 zeigt mögliche Subeinheiten, die Teil einer oder mehrerer der Steuereinrichtungen 5, insbesondere der Master-Steuereinrichtung, bilden können. Angedeutet ist in Fig. 4 zunächst ein Bildsystem 12, welches auch außerhalb der Steuereinrichtung 5 realisiert sein kann. Das Bildsystem 12 realisiert eine Bildverarbeitungskette, die es ermöglicht, mithilfe entsprechender Hardware- und/oder Softwarekomponenten eingehende Rohdaten zu einem Röntgen-Bilddatensatz zu verarbeiten. Auch optionale, weitere Bildverarbeitungsschritte können durch das Bildsystem 12 realisiert werden. Bezüglich der Übertragung von Bilddaten von (insbesondere anderen) Röntgeneinrichtungen 2a, 2b können Rohdaten erhalten werden, die dem Bildsystem 12 zugeführt werden, oder aber zumindest teilweise vorverarbeitete Bilddaten, die beispielsweise bereits im DICOM-Format vorliegen. Bei Bedarf, falls noch Bildverarbeitungsschritte durchzuführen sind, können auch derartige Bilddaten selbstverständlich an geeigneter Stelle in eine Bildverarbeitungskette des Bildsystems 12 eingespeist werden.

Fig. 4 zeigt ferner eine optionale Authentifizierungseinheit 13, die eine gegenseitige Authentifizierung von Kommunikationspartnern ermöglicht, gegebenenfalls auch unter Hinzunahme eines Benutzers, der beispielsweise eine PIN an einer Bedieneinrichtung 11 zumindest eines Kommunikationspartners eingibt, um die Kommunikationsverbindung und entsprechende Kopplung freizugeben.

Die Registrierungseinheit 14 kann auf verschiedene Arten für eine zumindest grobe Registrierung von Koordinatensystemen der einzelnen Röntgeneinrichtungen 2a, 2b sorgen. Eine grobe Registrierung kann beispielsweise dadurch hergestellt werden, dass eine mobile der Röntgeneinrichtungen 2a, 2b an eine bestimmte, durch einen Bodenmarker markierte Position in dem genutzten Raum bewegt wird und diese Positionierung bestätigt wird. Klare Bezugspunkte können bereits ausreichen, um Winkelstellungen von Aufnahmeanordnungen bzw. allgemeinen Aufnahmegeometrien in Relation zueinander zu setzen. Zur Registrierung können auch Positionsbestimmungssysteme optischer und/oder elektromagnetischer Art herangezogen werden, welche passive und/oder aktive Marker nutzen können, die zumindest teilweise auch an den Röntgeneinrichtungen 2a, 2b selbst vorgesehen sein können. Entsprechende, hier der Übersichtlichkeit halber nicht näher dargestellte Detektionseinrichtungen für die Marker können eingesetzt werden. Beispielsweise kann wenigstens eine mobile der Röntgeneinrichtungen 2a, 2b optische und/oder elektromagnetische Sensoren aufweisen, die Marker detektieren und somit Abstände zu anderen Merkmalen, insbesondere fest installierten Merkmalen des Raumes liefern, beispielsweise die Nutzung bestimmter Merkmale eines Patiententisches, beispielsweise an dessen Fuß, als Marker.

Ferner ist in Fig. 4 eine Synchronisationseinheit zur Synchronisierung von Zeitgebern der Röntgeneinrichtungen 2a, 2b dargestellt. Eine derartige Synchronisierung kann beispielsweise genutzt werden, um gleiche Pulsraten, gleiche Aufnahmezeitpunkte und dergleichen zu erreichen. Hier ist eine Ausgestaltung, in der eine Master-Steuereinrichtung verwendet wird, besonders zweckmäßig, da beispielsweise die Master-Steuereinrichtung ein Taktsignal vorgeben kann. Dabei sind verschiedene Synchronisierungsverfahren denkbar, beispielsweise die Nutzung einer softwarebasierten Synchronisierung und/oder die Verwendung von speziell für die Synchronisierung vorgesehenen Leitungen, die insbesondere mittels der Kommunikationsschnittstellen 4 angeschlossen werden können.

Fig. 5 zeigt ein erstes konkretes Ausführungsbeispiel einer erfindungsgemäßen Röntgenanordnung 1d. Diese weist vorliegend zwei mobile Röntgeneinrichtungen 16a, 16b, deren Mobilitätseinrichtung 17 vorliegend Räder 18 und auch eine zugeordnete, optionale Antriebsvorrichtung umfasst, die der Übersichtlichkeit halber nicht dargestellt ist. Beide Röntgeneinrichtungen 16a, 16b weisen einen Träger 19 mit jeweils einem C-Bogen 20 auf, an dem sich gegenüberliegend jeweils ein Röntgenstrahler 21 und ein Röntgendetektor 22 angeordnet sind. Jeweils ein Röntgenstrahler 21 und ein zugehöriger Röntgendetektor 22 bilden mithin eine Aufnahmeanordnung, die von den jeweiligen Trägern 19 getragen wird, wobei geeignete Verstelleinrichtungen 23 die automatisierte Einstellung verschiedener Aufnahmegeometrien erlauben, insbesondere auch wenigstens eine Rotation und/oder sonstige Verstellung des C-Bogens 20 und somit der Aufnahmeanordnung. Auch den Röntgenstrahlern 21 und/oder den Röntgendetektoren 22 können Verstelleinrichtungen 23 zugeordnet sein.

Die Verstelleinrichtungen 23 werden von den jeweiligen Steuereinrichtungen 5 der mobilen Röntgeneinrichtungen 16a, 16b angesteuert. Die Steuereinrichtungen 5 können mittels der über die Kommunikationsschnittstellen 4 hergestellten Kommunikationsverbindung 3, hier eine direkte drahtlose Kommunikationsverbindung 3, kommunizieren.

An den Trägern 19 der Röntgeneinrichtungen 16a, 16b sind vorliegend auch Darstellungseinrichtungen 10 sowie nicht näher dargestellte Bedieneinrichtungen 11 vorgesehen. Vorliegend kann eine Master-Steuereinrichtung der Steuereinrichtungen 5 beispielsweise durch einen Benutzer 24 gewählt werden, wobei auch automatische Auswahlverfahren bei den hier grundsätzlich gleichberechtigten mobilen Röntgeneinrichtungen 16a und 16b denkbar sind. Vorliegend wird der Benutzer 24 die Steuereinrichtung 5 der Röntgeneinrichtungen 16a, 16b als Master-Steuereinrichtung auswählen, von welcher aus er die Bedienung am einfachsten übernehmen kann.

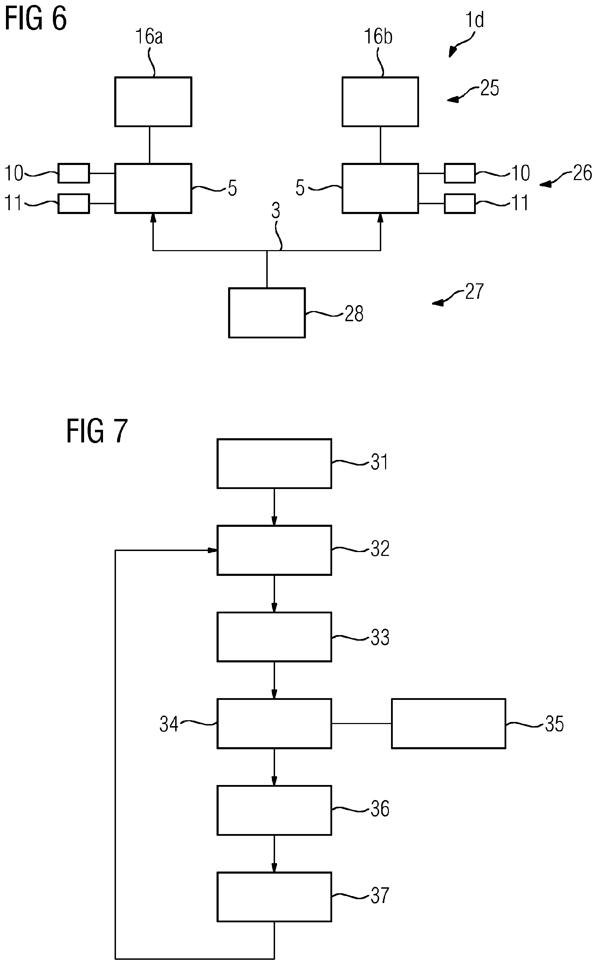

Fig. 6 zeigt die Röntgenanordnung 1d in einer Schichtstruktur schematisch nochmals. Eine Hardware-Schicht 25 wird durch die mobilen Röntgeneinrichtungen 16a, 16b, die auch als mobile C-Bögen bezeichnet werden können, bereitgestellt. In einer Steuerschicht 26 sind die Steuereinrichtungen 5 sowie die Darstellungseinrichtungen 10 sowie die Bedieneinrichtungen 11 dargestellt. In einer Datenschicht 27 werden über die Kommunikationsverbindung 3 Kommunikationsdaten 28 übertragen, welche vorliegend beispielhaft Steuerdaten, Bilddaten und Statusdaten, beispielsweise die aktuell eingestellte Aufnahmegeometrie, insbesondere einen Projektionswinkel, betreffend, umfassen.

Dabei sei an dieser Stelle angemerkt, dass mit der Bestimmung einer Steuereinrichtung 5 als Master-Steuereinrichtung vorzugsweise die Bedieneinrichtung 11 der anderen Röntgeneinrichtung 16a, 16b zumindest zeitweise deaktiviert werden, so dass mithin die Bedienung zentral von einer der Röntgeneinrichtungen 16a, 16b erfolgen kann. Die Darstellungseinrichtungen 10, beispielsweise umfassend wenigstens einen Monitor, können jedoch bei beiden Röntgeneinrichtungen 16a, 16b aktiv gehalten werden, so dass der Benutzer 24 von verschiedenen Positionen um den hier nur angedeuteten Patiententisch 29 (siehe Fig. 5, mit Patient 30) aufgenommene Bilddatensätze einsehen kann. Nachdem auch Bilddaten über die Kommunikationsverbindung 3 übermittelt werden können, können auf beiden Darstellungseinrichtungen 10 Bilddatensätze beider Röntgeneinrichtungen 16a, 16b zur Anzeige gebracht werden.

Der besondere Vorteil der hier gezeigten Ausgestaltung ist, dass zwei mobile Röntgeneinrichtungen 16a, 16b als eine Biplan-Einrichtung zusammenwirken können, insbesondere eine gekoppelte Bewegung der Aufnahmeanordnungen bei gleichzeitiger Bildakquisition erreicht werden kann. Wie dies beispielsweise vorgenommen werden kann, zeigt der Verfahrensablaufplan der Fig. 7. Dort werden in einem Schritt 31 zunächst die Röntgeneinrichtungen 16a, 16b zum Datenaustausch verbunden, das bedeutet, die vorliegend beispielhaft drahtlose Kommunikationsverbindung 3 wird hergestellt. In einem Schritt 32 werden die Winkelstellungen der Aufnahmeanordnungen für jeden der C-Bögen 20 getrennt eingestellt, so dass sich Winkeldifferenzen ergeben, beispielsweise bezüglich einer LAO/RAO-Drehachse und einer CRA/CAU-Drehachse. Diese Anpassung der Start-Aufnahmegeometrien kann manuell und/oder automatisch erfolgen. Zudem gilt, dass es aufgrund der bestehenden Kommunikationsverbindung 3 grundsätzlich bereits möglich ist, über eine Bedieneinrichtung 11 einer der Röntgeneinrichtungen 16a, 16b, insbesondere jener, deren Steuereinrichtung 5 die Master-Steuereinrichtung ist, beide Röntgeneinrichtungen 16a, 16b bzw. konkret deren Verstelleinrichtungen 23 getrennt anzusteuern. Dabei kann beispielsweise eine Benutzeroberfläche auf der jeweiligen Darstellungseinrichtung 10 angezeigt werden, in der die Auswahl der zu steuernden Röntgeneinrichtung 16a, 16b sowie konkrete Verstellkommandos eingegeben werden können.

Dabei sei an dieser Stelle angemerkt, dass bei optional auch automatisch verfahrbaren Röntgeneinrichtungen 16a, 16b, also solchen, deren Mobilitätseinrichtung 17 eine ansteuerbare Antriebsvorrichtung aufweist, auch die grundsätzliche Positionierung der Röntgeneinrichtungen 16a, 16b über eine zentrale Steuerung mittels der Master-Steuereinrichtung erfolgen kann.

In allen diesen Fällen werden von der Steuereinrichtung 5, die als Master-Steuereinrichtung gewählt ist, Steuerdaten über die Kommunikationsverbindung 3 an die andere Steuereinrichtung 5 übertragen, wo sie entsprechend von dieser als Slave-Steuereinrichtung gewählten Steuereinrichtung 5 umgesetzt werden, um die Verstelleinrichtungen 23 sowie gegebenenfalls die Antriebsvorrichtung anzusteuern.

Wie bereits erwähnt, ist das Resultat des Schrittes 32 eine bestimmte Einstellung von relativen Aufnahmegeometrien der Aufnahmeanordnungen der Röntgeneinrichtungen 16a, 16b, vorliegend unterschiedliche Drehwinkel der zwei Drehachsen und somit Winkeldifferenzen in diesen zwei Drehachsen.

In einem Schritt 33 kann dann seitens des Benutzers 24, beispielsweise in einem Benutzerinterface auf der Darstellungseinrichtung 10, eine Bewegungskopplung der Aufnahmeanordnungen angewählt werden, wobei es spätestens zu diesem Zeitpunkt zweckmäßig ist, die Bedieneinrichtung 11 der Slave-Röntgeneinrichtung 16a, 16b zu deaktivieren. In einem Schritt 34 erfolgt dann die koordinierte Steuerung der Röntgeneinrichtungen 16a, 16b zum Bildaufnahmebetrieb, was sowohl durch ein vorgegebenes Messprogramm als auch aufgrund von manuellen Bedieneingaben des Benutzers 24 möglich ist. Dabei werden im Schritt 34 die Winkeldifferenzen zwischen den Aufnahmegeometrien immer aufrechterhalten, das bedeutet, jede Bewegung einer der Aufnahmeanordnungen resultiert in einer automatischen Ansteuerung der Verstelleinrichtungen 23 für die andere Aufnahmeanordnung derart, dass die Winkelunterschiede wie ursprünglich zum Zeitpunkt der Bewegungskopplung eingestellt aufrechterhalten werden. Parallel können im Bildaufnahmebetrieb in einem Schritt 35 aufgenommene Bilddatensätze der Röntgeneinrichtungen 16a, 16b auf den Darstellungseinrichtungen 10 dargestellt werden.

In einem Schritt 36 wird die Bewegungskopplung der Röntgeneinrichtungen 16a, 16b, beispielsweise wiederum über ein Bedienelement der Benutzerschnittstelle, wieder aufgehoben. Optional können nun in einem Schritt 37 wieder unabhängige Verstellungen bzw. Bewegungen der Röntgeneinrichtungen 16a, 16b vorgenommen werden, so dass beispielsweise die relativen Aufnahmegeometrien neu eingestellt werden können und wieder mit Schritt 32 fortgefahren werden kann.

Dabei sei hervorgehoben, dass das in Fig. 7 anhand des einfachen Beispiels wenigstens eines aufrechterhaltenen Relativwinkels zwischen den Aufnahmegeometrien beschriebene Vorgehen selbstverständlich auch auf andere koordinierte Steuerungsansätze übertragen werden kann, die sich mittels einer zentralen Steuerung ergeben können. Insbesondere bei vollständiger Registrierung der Koordinatensysteme der Röntgeneinrichtungen 16a, 16b können komplette, koordinierte Bewegungsabläufe vorausgeplant werden; auch andere Aufnahmeparameter können koordiniert angepasst werden, so dass beispielsweise beide Röntgeneinrichtungen 16a, 16b mit unterschiedlichen Röntgenspektren messen können.

Hinsichtlich der Registrierung sei an dieser Stelle im Übrigen noch angemerkt, dass, vgl. Fig. 4, wenigstens eine der Steuereinrichtungen 5 auch eine Kollisionsvermeidungseinheit 38 aufweisen kann, so dass, insbesondere bei bestehender Registrierung mittels der Registrierungseinheit 14, über die Kommunikationsverbindung 3 erhaltene Positionsdaten von Komponenten der anderen Röntgeneinrichtung 16a, 16b ebenso für eine Gesamt-Kollisionsvermeidung berücksichtigt werden können.

Das bedeutet insgesamt, dass letztlich eine tiefe Integration stattfindet, die es erlaubt, bei Bedarf die auch einzeln verwendbaren Röntgeneinrichtungen 16a und 16b als ein voll funktionsfähiges Biplan-Röntgengerät zusammenzufassen, nachdem vorliegend die Slave-Röntgeneinrichtung 16a, 16b hinsichtlich der Bedienung, der Kollisionsbetrachtung und der Steuerung, insbesondere sowohl bezüglich Strahlungskette als auch bezüglich Bildkettet, vollständig inkludiert ist.

Es sei angemerkt, dass sich diese Ausführungen genau wie die zum Verfahren gemäß Fig. 7 und den dargelegten verallgemeinerten Einsatzmöglichkeiten selbstverständlich analog auf die noch folgenden Ausführungsbeispiele von Röntgenanordnungen übertragen lassen.

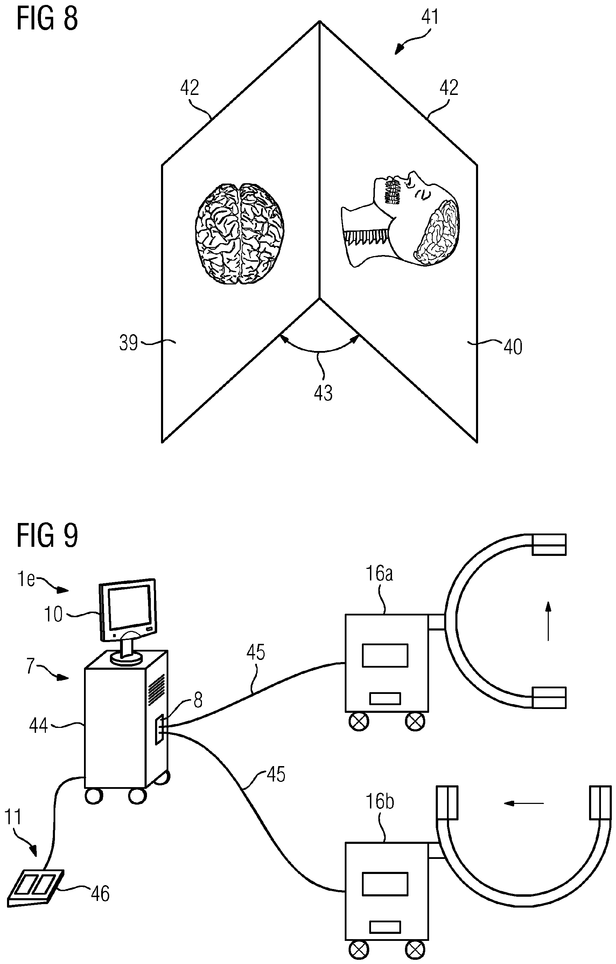

Zunächst zeigt jedoch Fig. 8 eine Möglichkeit zur Visualisierung von zweidimensionalen Bilddatensätzen, hier Projektionsbildern 39, 40 der unterschiedlichen Röntgeneinrichtungen 16a, 16b, die gleichzeitig aufgenommen wurden. Aufgrund der unterschiedlichen Winkelstellung der Aufnahmeanordnungen liegen unterschiedliche Projektionswinkel für die Projektionsbilder 39, 40 vor. Die Darstellung auf der Darstellungseinrichtung 10 erfolgt nun nach Art eines Buches 41, dessen Seiten 42 von den Projektionsbildern 39, 40 gebildet werden. Die Seiten 42 sind so gegeneinander aufgestellt, dass deren dargestellter Öffnungswinkel 43 der Winkeldifferenz zwischen den Projektionswinkeln der Projektionsbilder 39, 40 (und somit der Winkeldifferenz zwischen den Aufnahmeanordnungen) entspricht. Die entsprechenden Projektionswinkel können gegebenenfalls auch in die Projektionsbilder 39, 40 eingeblendet werden, wie hier mit beispielhaften Werten (0°, 90°) vorgesehen. Sollen weitere Projektionsbilder der Visualisierung hinzugefügt werden, können weitere Seiten 42 des Buches 41 in den entsprechenden Winkelabständen ergänzt werden; mittels geeigneter Bedienelemente an der Bedieneinrichtung 11 bzw. bei Ausbildung der Darstellungseinrichtung 10 als ein Touchscreen auf der Darstellungseinrichtung 10 kann ein Benutzer 24 durch das Buch 41 "hindurchblättern".

Auch unabhängig von den hier dargestellten Röntgenanordnungen 1a bis 1f lässt sich dieses Visualisierungsverfahren nützlich einsetzen, beispielsweise auch bei der Magnetresonanzbildgebung und dergleichen.

Fig. 9 zeigt ein weiteres konkretes Ausführungsbeispiel einer Röntgenanordnung 1e, die vorliegend eine als Monitorwagen 44 ausgebildete Zwischeneinrichtung 7 aufweist, an die die beiden mobilen Röntgeneinrichtungen 16a, 16b vorliegend über drahtgebundene Kabel 45 angeschlossen sind. Die Kommunikationsschnittstelle 8 der Zwischeneinrichtung 7 weist entsprechend wenigstens zwei Anschlüsse 9, insbesondere Anschlussbuchsen, auf. Als weitere, häufig eingesetzte Bedieneinrichtung 11 (bzw. als Bedienelement) ist vorliegend ein Fußschalter 46 gezeigt, der, nachdem die Steuereinrichtung 5 der Zwischeneinrichtung 7 als Master-Steuereinrichtung verwendet wird, zur Bedienung beider Röntgeneinrichtungen 16a, 16b genutzt werden kann.

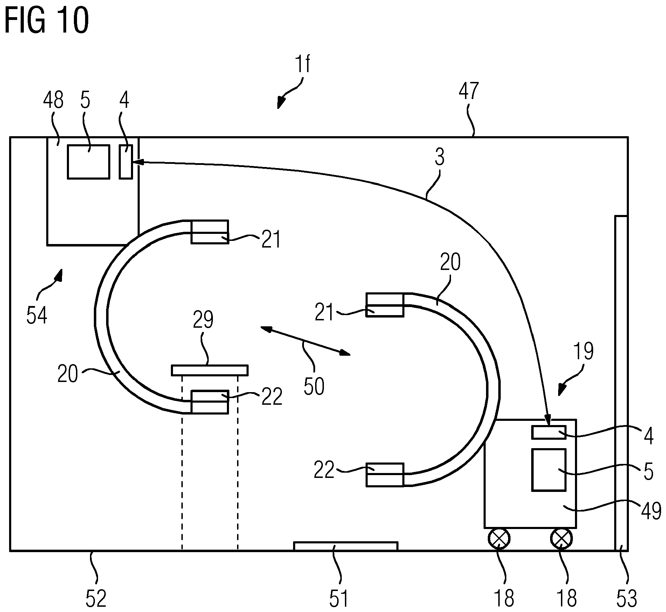

Fig. 10 zeigt eine besonders bevorzugte dritte konkrete Ausgestaltung einer Röntgenanordnung 1f. Diese ist in einem Raum 47, in dem sie genutzt werden soll, beispielsweise einem Operationsraum als Behandlungsraum, gezeigt. In dem Raum 47 befindet sich wiederum ein Patiententisch 29.

Eine erste, fest installierte Röntgeneinrichtung 48 weist einen an der Decke montierten Träger 54 mit einem C-Bogen 20 auf, wobei eine zweite Röntgeneinrichtung 49 vergleichbar wie die Röntgeneinrichtungen 16a, 16b ausgebildet ist. Die Röntgeneinrichtung 49 ist mithin mobil und kann gegen die fest installierte Röntgeneinrichtung 48 beliebig bewegt werden, wie durch den Pfeil 50 angedeutet wird. Auch die Röntgeneinrichtung 49 weist einen Träger 19 mit einem C-Bogen 20 auf, an dem als Aufnahmeanordnung sich gegenüberliegend ein Röntgenstrahler 21 und ein Röntgendetektor 22 angeordnet sind. Über Kommunikationsschnittstellen 4 kann wiederum direkt oder indirekt die Kommunikationsverbindung 3 hergestellt werden, bevorzugt die in den grundlegenden Ausgestaltungen der Fig. 1 oder Fig. 2 dargestellt.

Fig. 10 zeigt auch beispielhaft einen Marker 51 am Boden 52 des Raumes 47, der beispielsweise zur geeigneten, insbesondere initialen, Positionierung der Röntgeneinrichtung 49 genutzt werden kann.

Gemäß der bezüglich anderer Ausführungsbeispiele bereits dargelegten Ausgestaltungen kann aufgrund der kommunikativen Ankopplung der Röntgeneinrichtung 49 an die Röntgeneinrichtung 48 ein voll funktionsfähiges Biplan-Röntgengerät geschaffen werden, bei welchem mithin zwei Ebenen zur Aufnahme bereitstehen. Nichtsdestotrotz können beide Röntgeneinrichtungen 48, 49 auch vollständig unabhängig voneinander genutzt werden, wenn nur eine Ebene benötigt wird. Dabei kann beispielsweise die Röntgeneinrichtung 49, die ja mobil ist, beispielsweise durch eine Tür 53 oder dergleichen in einen benachbarten Raum verbracht werden, so dass beide Röntgeneinrichtungen 48, 49 unabhängig voneinander auch parallel eingesetzt werden können. Hierdurch ergibt sich eine hohe Flexibilität. Für beispielsweise neurologische Anwendungen, die eine zweite Ebene benötigen, kann der mobile C-Bogen 20 der Röntgeneinrichtung 49 angekoppelt werden.

Dabei sei darauf hingewiesen, dass zusätzlich zu der deckenmontierten Röntgeneinrichtung 48 und/oder alternativ zu dieser in Fig. 10 auch eine Sliding Gantry-CT-Einrichtung verwendet werden kann, so dass dann insbesondere auch drei Röntgeneinrichtungen einer Röntgenanordnung vorliegen können.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Röntgenanordnung (1a, 1b, 1c, 1d, 1e, 1f), umfassend wenigstens zwei Röntgeneinrichtungen (2a, 2b, 16a, 16b, 48, 49) mit jeweils einer Steuereinrichtung (5) und einer einen Röntgenstrahler (21) und einen Röntgendetektor (22) aufweisenden Aufnahmeanordnung, wobei wenigstens eine der Röntgeneinrichtungen (2a, 2b, 16a, 16b, 48, 49) eine mobile Röntgeneinrichtung (2a, 2b, 16a, 16b, 49) mit einem mobilen, die Aufnahmeanordnung tragenden Träger (19) ist, wobei die Röntgeneinrichtungen (2a, 2b, 16a, 16b, 48, 49) jeweils eine Kommunikationsschnittstelle (4) zur Herstellung einer Kommunikationsverbindung (3) zwischen den Steuereinrichtungen (5) der Röntgeneinrichtungen (2a, 2b, 16a, 16b, 48, 49) und/oder zwischen einer zur Röntgenanordnung (1a, 1b, 1c, 1d, 1e, 1f) gehörigen Zwischeneinrichtung (7), die selbst eine Steuereinrichtung (5) und wenigstens eine Kommunikationsschnittstelle (8) aufweist, und allen Steuereinrichtungen (5) der Röntgeneinrichtungen (2a, 2b, 16a, 16b, 48, 49) aufweisen, wobei die Steuereinrichtung (5) wenigstens einer der wenigstens einen mobilen Röntgeneinrichtung (2a, 2b, 16a, 16b, 49) zur Steuerung des Bildaufnahmebetriebs der jeweiligen Röntgeneinrichtung (2a, 2b, 16a, 16b, 48, 49) anhand von über die Kommunikationsverbindung (3) von einer anderen Steuereinrichtung (5) erhaltenen Steuerdaten und/oder zur Übermittlung wenigstens eines aufgenommenen Bilddatensatzes an wenigstens eine weitere Steuereinrichtung (5) über die Kommunikationsverbindung (3) ausgebildet ist.

2. Röntgenanordnung (1a, 1b, 1c, 1d, 1e, 1f) nach Anspruch 1, **dadurch gekennzeichnet, dass** die Kommunikationsschnittstellen (4, 8) zur drahtlosen Kommunikation ausgebildet sind und/oder der Träger (19) einen C-Bogen (20) umfasst.

3. Röntgenanordnung (1a, 1b, 1c, 1d, 1e, 1f) nach Anspruch 1 oder 2, **dadurch gekennzeichnet, dass** eine als Master ausgebildete oder benutzerseitig und/oder automatisch als Master bestimmte Steuereinrichtung (5) zur koordinierten Steuerung der gesamten Röntgenanordnung (1a, 1b, 1c, 1d, 1e, 1f) ausgebildet ist.

4. Röntgenanordnung (1a, 1b, 1c, 1d, 1e, 1f) nach Anspruch 3, **dadurch gekennzeichnet, dass** den Aufnahmeanordnungen mittels der jeweiligen Steuereinrichtungen (5) ansteuerbare Verstelleinrichtungen (23) zur Einstellung unterschiedlicher Aufnahmegeometrien zugeordnet sind, wobei die als Master dienende Steuereinrichtung (5) zur bewegungskoppelnden Ansteuerung der Verstelleinrichtungen (23) von wenigstens zwei der wenigstens zwei Röntgeneinrichtungen (2a, 2b, 16a, 16b, 48, 49) ausgebildet ist.

5. Röntgenanordnung (1a, 1b, 1c, 1d, 1e, 1f) nach einem der vorangehenden Ansprüche, **dadurch gekennzeichnet, dass** wenigstens eine Steuereinrichtung (5), insbesondere wenigstens die als Master ausgebildete Steuereinrichtung (5), eine Registrierungseinheit (14) zur Registrierung von Koordinatensystemen der unterschiedlichen Röntgeneinrichtungen (2a, 2b, 16a, 16b, 48, 49) aufweist.

6. Röntgenanordnung (1a, 1b, 1c, 1d, 1e, 1f) nach Anspruch 5, **dadurch gekennzeichnet, dass** wenigstens eine einer Röntgeneinrichtung (2a, 2b, 16a, 16b, 48, 49) zumindest zugeordnete Steuereinrichtung (5) eine Kollisionsvermeidungseinheit (38) für diese Röntgeneinrichtung (2a, 2b, 16a, 16b, 48, 49) aufweist, wobei die Kollisionsvermeidungseinheit (38) zur Berücksichtigung von über die Kommunikationsverbindung (3) erhaltenen Positionsinformationen wenigstens einer weiteren Röntgeneinrichtung (2a, 2b, 16a, 16b, 48, 49) ausgebildet ist.

7. Röntgenanordnung (1a, 1b, 1c, 1d, 1e, 1f) nach Anspruch 5 oder 6, **dadurch gekennzeichnet, dass** wenigstens eine Steuereinrichtung (5), insbesondere die als Master ausgebildete Steuereinrichtung (5), zur Ermittlung von eine vorbestimmte relative Positionierung wenigstens der Aufnahmeanordnungen von wenigstens zwei der wenigstens zwei Röntgeneinrichtungen (2a, 2b, 16a, 16b, 48, 49) herstellenden Anweisungsdaten ausgebildet ist, welche zur manuellen Einstellung über eine Darstellungseinrichtung (10) ausgebbar und/oder wenigstens teilweise zur Ansteuerung von der Positionsanpassung dienenden Komponenten wenigstens einer Röntgeneinrichtung (2a, 2b, 16a, 16b, 48, 49) verwendet werden, insbesondere nach einer Übertragung über wenigstens eine der Kommunikationsverbindungen (3) .

8. Röntgenanordnung (1a, 1b, 1c, 1d, 1e, 1f) nach einem der vorangehenden Ansprüche, **dadurch gekennzeichnet, dass** wenigstens eine der Steuereinrichtungen (5) eine Synchronisationseinheit (15) zur Synchronisierung von Zeitgebern von wenigstens zwei über wenigstens der wenigstens einen Kommunikationsverbindung (3) verbundenen Röntgeneinrichtungen (2a, 2b, 16a, 16b, 48, 49) aufweist.

9. Röntgenanordnung (1a, 1b, 1c, 1d, 1e, 1f) nach einem der vorangehenden Ansprüche, **dadurch gekennzeichnet, dass** sie ferner wenigstens einen fest installierten Generator und/oder wenigstens eine fest installierte Kühleinrichtung mit Anschlüssen (9) für die wenigstens eine mobile Röntgeneinrichtung (2a, 2b, 16a, 16b, 49) aufweist.

10. Röntgenanordnung (1a, 1b, 1c, 1d, 1e, 1f) nach einem der vorangehenden Ansprüche, **dadurch gekennzeichnet, dass** wenigstens eine der Röntgeneinrichtungen (2a, 2b, 16a, 16b, 48, 49), insbesondere eine fest installierte Röntgeneinrichtung (2a, 2b, 48), ein Bildsystem (12) aufweist, wobei wenigstens eine weitere der Röntgeneinrichtungen (2a, 2b, 16a, 16b, 48, 49), insbesondere eine mobile Röntgeneinrichtung (2a, 2b, 16a, 16b, 49), zur Mitnutzung des Bildsystems (12) ausgebildet ist, insbesondere durch Übertragung von Bilddaten über die Kommunikationsverbindung (3).

11. Röntgenanordnung (1a, 1b, 1c, 1d, 1e, 1f) nach Anspruch 10, **dadurch gekennzeichnet, dass** das Bildsystem (12) zur gemeinsamen Auswertung von Bilddaten mehrerer Röntgeneinrichtungen (2a, 2b, 16a, 16b, 48, 49) ausgebildet ist.

12. Röntgenanordnung (1a, 1b, 1c, 1d, 1e, 1f) nach einem der vorangehenden Ansprüche, **dadurch gekennzeichnet, dass** die Röntgenanordnung (1a, 1b, 1c, 1d, 1e, 1f) wenigstens eine Darstellungseinrichtung (10) für mit den Röntgeneinrichtungen (2a, 2b, 16a, 16b, 48, 49) aufgenommene Bilddatensätze aufweist.

13. Röntgenanordnung (1a, 1b, 1c, 1d, 1e, 1f) nach Anspruch 12, **dadurch gekennzeichnet, dass** die Darstellungseinrichtung (10) zur Darstellung von unter unterschiedlichen Projektionswinkeln, insbesondere mit unterschiedlichen Röntgeneinrichtungen (2a, 2b, 16a, 16b, 48, 49), aufgenommenen zweidimensionalen Projektionsbildern (39, 40) als Bilddatensätze gemeinsam in einer 3D-Visualisierung nach Art eines Buches (41) ausgebildet ist, wobei jede dargestellte Seite (42) des Buches (41) einem Projektionsbild (39, 40) entspricht und die Seiten (42) gegeneinander in einem Winkel (43), der dem Projektionswinkelunterschied der jeweiligen Projektionsbilder (39, 40) entspricht, aufgestellt sind.

14. Röntgenanordnung (1a, 1b, 1c, 1d, 1e, 1f) nach einem der vorangehenden Ansprüche, **dadurch gekennzeichnet, dass** eine der Röntgeneinrichtungen (2a, 2b, 16a, 16b, 48, 49) eine fest installierte, deckengehängte Röntgeneinrichtung (48) mit einem C-Bogen (20), der die Aufnahmeanordnung trägt, oder eine Sliding Gantry-CT-Einrichtung ist, wobei deren Bedien- und/ oder Darstellungseinrichtungen (11, 10) aufgrund der wenigstens einen Kommunikationsverbindung (3) auch zur Nutzung mit der wenigstens einen mobilen Röntgeneinrichtung (2a, 2b, 16a, 16b, 49) ausgebildet sind.

15. Verfahren zum Betrieb einer Röntgenanordnung (1a, 1b, 1c, 1d, 1e, 1f), umfassend wenigstens zwei Röntgeneinrichtungen (2a, 2b, 16a, 16b, 48, 49) mit jeweils einer Steuereinrichtung (5) und einer einen Röntgenstrahler (21) und einen Röntgendetektor (22) aufweisenden Aufnahmeanordnung, wobei wenigstens eine der Röntgeneinrichtungen (2a, 2b, 16a, 16b, 48, 49) eine mobile Röntgeneinrichtung (2a, 2b, 16a, 16b, 49) mit einem mobilen, die Aufnahmeanordnung tragenden Träger (19) ist, wobei mittels jeweils einer Kommunikationsschnittstelle (4) der Röntgeneinrichtungen (2a, 2b, 16a, 16b, 48, 49) eine Kommunikationsverbindung (3) zwischen den Steuereinrichtungen (5) der Röntgeneinrichtungen (2a, 2b, 16a, 16b, 48, 49) und/oder zwischen einer zur Röntgenanordnung (1a, 1b, 1c, 1d, 1e, 1f) gehörigen Zwischeneinrichtung (7), die selbst eine Steuereinrichtung (5) und wenigstens eine Kommunikationsschnittstelle (8) aufweist, und allen Steuereinrichtungen (5) der Röntgeneinrichtungen (2a, 2b, 16a, 16b, 48, 49) hergestellt wird, wobei die Steuereinrichtung (5) wenigstens einer der wenigstens einen mobilen Röntgeneinrichtung (2a, 2b, 16a, 16b, 49) den Bildaufnahmebetriebs der jeweiligen Röntgeneinrichtung (2a, 2b, 16a, 16b, 48, 49) anhand von über die Kommunikationsverbindung (3) von einer anderen Steuereinrichtung (5) erhaltenen Steuerdaten steuert und/oder wenigstens einen aufgenommenen Bilddatensatz an wenigstens eine weitere Steuereinrichtung (5) über die Kommunikationsverbindung (3) übermittelt.

## Claims

1. X-ray assembly (1a, 1b, 1c, 1d, 1e, 1f) including at least two X-ray facilities (2a, 2b, 16a, 16b, 48, 49) each with a control facility (5) and a recording assembly comprising an X-ray tube (21) and an X-ray detector (22), wherein at least one of the X-ray facilities (2a, 2b, 16a, 16b, 48, 49) is a mobile X-ray facility (2a, 2b, 16a, 16b, 49) with a mobile carrier (19) carrying the recording assembly, wherein the X-ray facilities (2a, 2b, 16a, 16b, 48, 49) each comprise a communication interface (4) for establishing a communication connection (3) between the control facilities (5) of the X-ray facilities (2a, 2b, 16a, 16b, 48, 49) and/or between an intermediate facility (7) belonging to the X-ray assembly (1a, 1b, 1c, 1d, 1e, If), which itself comprises a control facility (5) and at least one communication interface (8), and all the control facilities (5) of the X-ray facilities (2a, 2b, 16a, 16b, 48, 49), wherein the control facility (5) of least one of the at least one mobile X-ray facility (2a, 2b, 16a, 16b, 49) is embodied to control the image recording operation of the respective X-ray facility (2a, 2b, 16a, 16b, 48, 49) using control data received from another control facility (5) via the communication connection (3) and/or to transfer at least one recorded image dataset to at least one further control facility (5) via the communication connection (3).

2. X-ray assembly (1a, 1b, 1c, 1d, 1e, 1f) according to claim 1, **characterised in that** the communication interfaces (4, 8) are embodied for wireless communication and/or the carrier (19) includes a C-arm (20).

3. X-ray assembly (1a, 1b, 1c, 1d, 1e, 1f) according to claim 1 or 2, **characterised in that** a control facility (5) embodied as the master or specified as the master by the user and/or automatically is embodied for the coordinated control of the entire X-ray assembly (1a, 1b, 1c, 1d, 1e, 1f).

4. X-ray assembly (1a, 1b, 1c, 1d, 1e, 1f) according to claim 3, **characterised in that** the recording assemblies are assigned adjusting facilities (23) that can be actuated by means of respective control facilities (5) for setting different recording geometries, wherein the control facility (5) serving as the master is embodied to actuate the adjusting facilities (23) of at least two of the at least two X-ray facilities (2a, 2b, 16a, 16b, 48, 49) with kinematic coupling.

5. X-ray assembly (1a, 1b, 1c, 1d, 1e, 1f) according to one of the preceding claims, **characterised in that** at least one control facility (5), in particular at least the control facility (5) embodied as the master, comprises a registration unit (14) for registering coordinate facilities of the different X-ray facilities (2a, 2b, 16a, 16b, 48, 49).

6. X-ray assembly (1a, 1b, 1c, 1d, 1e, 1f) according to claim 5, **characterised in that** at least one control facility (5) assigned to at least one X-ray facility (2a, 2b, 16a, 16b, 48, 49) comprises a collision-avoiding unit (38) for said X-ray facility (2a, 2b, 16a, 16b, 48, 49), wherein the collision-avoiding unit (38) is embodied to take account of positional information of at least one further X-ray facility (2a, 2b, 16a, 16b, 48, 49) received via the communication connection (3) .

7. X-ray assembly (1a, 1b, 1c, 1d, 1e, 1f) according to claim 5 or 6, **characterised in that** at least one control facility (5), in particular the control facility (5) embodied as the master is embodied to determine instruction data establishing a prespecified relative positioning of at least the recording assemblies of at least two of the at least two X-ray facilities (2a, 2b, 16a, 16b, 48, 49), wherein said instruction data can be output for manual setting via a display facility (10) and/or is at least partially used to actuate components serving for positional adjustment of at least one X-ray facility (2a, 2b, 16a, 16b, 48, 49), in particular after transmission via at least one of the communication connections (3).

8. X-ray assembly (1a, 1b, 1c, 1d, 1e, 1f) according to one of the preceding claims, **characterised in that** at least one of the control facilities (5) comprises a synchronisation unit (15) for synchronising timers of at least two X-ray facilities (2a, 2b, 16a, 16b, 48, 49) connected via at least the at least one communication connection (3).

9. X-ray assembly (1a, 1b, 1c, 1d, 1e, 1f) according to one of the preceding claims, **characterised in that** it further comprises at least one permanently installed generator and/or at least one permanently installed cooling facility with connectors (9) for the at least one mobile X-ray facility (2a, 2b, 16a, 16b, 49).

10. X-ray assembly (1a, 1b, 1c, 1d, 1e, 1f) according to one of the preceding claims, **characterised in that** at least one of the X-ray facilities (2a, 2b, 16a, 16b, 48, 49), in particular a permanently installed X-ray facility (2a, 2b, 48), comprises an imaging system (12), wherein at least one further one of the X-ray facilities (2a, 2b, 16a, 16b, 48, 49), in particular a mobile X-ray facility (2a, 2b, 16a, 16b, 49), is embodied for the common use of the imaging system (12), in particular by the transmission of image data via the communication connection (3).

11. X-ray assembly (1a, 1b, 1c, 1d, 1e, 1f) according to claim 10, **characterised in that** the imaging system (12) is embodied for the common evaluation of image data from a plurality of X-ray facilities (2a, 2b, 16a, 16b, 48, 49).

12. X-ray assembly (1a, 1b, 1c, 1d, 1e, 1f) according to one of the preceding claims, **characterised in that** the X-ray assembly (1a, 1b, 1c, 1d, 1e, 1f) comprises at least one depiction facility (10) for image datasets recorded with the X-ray facilities (2a, 2b, 16a, 16b, 48, 49).

13. X-ray assembly (1a, 1b, 1c, 1d, 1e, 1f) according to claim 12, **characterised in that** the depiction facility (10) is embodied for the depiction of two-dimensional projection images (39, 40) recorded under different projection angles, in particular with different X-ray facilities (2a, 2b, 16a, 16b, 48, 49), as image datasets jointly in a 3D visualisation in the style of a book (41), wherein each page (42) of the book (41) depicted corresponds to a projection image (39, 40) and the pages (42) are presented with respect to one another at an angle (43) corresponding to the difference in the projection angles of the respective projection images (39, 40).

14. X-ray assembly (1a, 1b, 1c, 1d, 1e, 1f) according to one of the preceding claims, **characterised in that** one of the X-ray facilities (2a, 2b, 16a, 16b, 48, 49) is a permanently installed ceiling-suspended X-ray facility (48) with a C-arm (20), which carries the recording assembly, or a sliding-gantry CT facility, wherein, due to the at least one communication connection (3), the operating and/or depiction facilities (11, 10) are also embodied for use with the at least one mobile X-ray facility (2a, 2b, 16a, 16b, 49).

15. Method for operating an X-ray assembly (1a, 1b, 1c, 1d, 1e, 1f) including at least two X-ray facilities (2a, 2b, 16a, 16b, 48, 49) each comprising a control facility (5) and a recording assembly comprising an X-ray tube (21) and an X-ray detector (22), wherein at least one of the X-ray facilities (2a, 2b, 16a, 16b, 48, 49) is a mobile X-ray facility (2a, 2b, 16a, 16b, 49) with a mobile carrier carrying the recording assembly carrier (19), wherein by means of in each case one communication interface (4) of the X-ray facilities (2a, 2b, 16a, 16b, 48, 49) a communication connection (3) is established between the control facilities (5) of the X-ray facilities (2a, 2b, 16a, 16b, 48, 49) and/or between an intermediate facility (7) belonging to the X-ray assembly (1a, 1b, 1c, 1d, 1e, If), said intermediate facility itself comprising a control facility (5), and at least one communication interface (8), and all the control facilities (5) of the X-ray facilities (2a, 2b, 16a, 16b, 48, 49), wherein the control facility (5) of least one of the at least one mobile X-ray facility (2a, 2b, 16a, 16b, 49) controls the image recording operation of the respective X-ray facility (2a, 2b, 16a, 16b, 48, 49) using control data received from another control facility (5) via the communication connection (3) and/or transfers at least one recorded image dataset to at least one further control facility (5) via the communication connection (3).

## Revendications

1. Système (1a, 1b, 1c, 1d, 1e, 1f) à rayons X, comprenant au moins deux dispositifs (2a, 2b, 16a, 16b, 48, 49) à rayons X, ayant chacun un dispositif (5) de commande et un système d'enregistrement ayant un émetteur (21) de rayons X et un détecteur (22) de rayons X, dans lequel au moins l'un des dispositifs (2a, 2b, 16a, 16b, 48, 49) à rayons X est un dispositif (2a, 2b, 16a, 16b, 49) à rayons X mobile ayant un support (19) mobile portant le système d'enregistrement, dans lequel
les dispositifs (2a, 2b, 16a, 16b, 48, 49) à rayons X ont chacun une interface (4) de communication pour établir une liaison (3) de communication entre les dispositifs (5) de commande des dispositifs (2a, 2b, 16a, 16b, 48, 49) à rayons X et/ou entre un dispositif (7) intermédiaire appartenant au système (1a, 1b, 1c, 1d, 1e, 1f) à rayons X, qui a soi-même un dispositif (5) de commande, et au moins une interface (8) de communication, et tous les dispositifs (5) de commande des dispositifs (2a, 2b, 16a, 16b, 48, 49) à rayons X, le dispositif (5) de commande d'au moins l'un du au moins un dispositif (2a, 2b, 16a, 16b, 49) à rayons X mobiles étant constitué pour commander le fonctionnement d'enregistrement d'image du dispositif (2a, 2b, 16a, 16b, 48, 49) à rayons X respectif, à l'aide de données de commande obtenues d'un autre dispositif (5) de commande par la liaison (3) de communication, et/ou pour la transmission d'au moins un ensemble de données d'image, enregistré à au moins un autre dispositif (5) de commande, par la liaison (3) de communication.

2. Système (1a, 1b, 1c, 1d, 1e, 1f) à rayons X suivant la revendication 1, **caractérisé en ce que** les interfaces (4, 8) de communication sont constituées pour la communication sans fil et/ou le support (19) comprend un arceau (20) en C.

3. Système (1a, 1b, 1c, 1d, 1e, 1f) à rayons X suivant la revendication 1 ou 2, **caractérisé en ce qu'**un dispositif (5) de commande constitué en maître ou déterminé, par l'utilisateur et/ou automatiquement, comme maître est constitué pour la commande coordonnée de l'ensemble de l'agencement (1a, 1b, 1c, 1d, 1e, 1f) à rayons X.

4. Système (1a, 1b, 1c, 1d, 1e, 1f) à rayons X suivant la revendication 3, **caractérisé en ce qu'**aux systèmes d'enregistrement sont associés, pour l'établissement de géométries d'enregistrement différentes, des dispositifs (23) de déplacement pouvant être commandés au moyen des dispositifs (5) de commande respectifs, le dispositif (5) de commande servant de maître étant constitué pour la commande couplée en déplacement des dispositifs (23) de déplacement d'au moins deux des au moins deux dispositifs (2a, 2b, 16a, 16b, 48, 49) à rayons X.

5. Système (1a, 1b, 1c, 1d, 1e, 1f) à rayons X suivant l'une des revendications précédentes, **caractérisé en ce qu'**au moins un dispositif (5) de commande, notamment au moins le dispositif (5) de commande constitué en maître, a une unité (14) d'enregistrement pour enregistrer des systèmes de coordonnées des différents dispositifs (2a, 2b, 16a, 16b, 48, 49) à rayons X.

6. Système (1a, 1b, 1c, 1d, 1e, 1f) à rayons X suivant la revendication 5, **caractérisé en ce qu'**au moins un dispositif (5) de commande associé au moins à un dispositif (2a, 2b, 16a, 16b, 48, 49) à rayons X a une unité (38) empêchant une collision pour ce dispositif (2a, 2b, 16a, 16b, 48, 49) à rayons X, l'unité (38) empêchant une collision étant constituée pour prendre en compte des informations de position, obtenue par la liaison (3) de communication, d'au moins un autre dispositif (2a, 2b, 16a, 16b, 48, 49) à rayons X.

7. Système (1a, 1b, 1c, 1d, 1e, 1f) à rayons X suivant la revendication 5 ou 6, **caractérisé en ce qu'**au moins un dispositif (5) de commande, notamment le dispositif (5) de commande constitué en maître, est constitué pour la détermination de données d'instruction donnant un positionnement relatif déterminé à l'avance d'au moins les systèmes d'enregistrement d'au moins deux des au moins deux dispositifs (2a, 2b, 16a, 16b, 48, 49) à rayons X, données qui, pour le réglage manuel, peuvent être sorties par un dispositif (10) de représentation et/ou être utilisées, au moins en partie, pour commander des composants, servant à l'adaptation de position, d'au moins un dispositif (2a, 2b, 16a, 16b, 48, 49) à rayons X, notamment après une transmission par au moins l'une des liaisons (3) de communication.

8. Système (1a, 1b, 1c, 1d, 1e, 1f) à rayons X suivant l'une des revendications précédentes, **caractérisé en ce qu'**au moins l'un des dispositifs (5) de commande a une unité (15) de synchronisation pour la synchronisation d'horloges d'au moins deux dispositifs (2a, 2b, 16a, 16b, 48, 49) à rayons X reliés par au moins la au moins une liaison (3) de communication.

9. Système (1a, 1b, 1c, 1d, 1e, 1f) à rayons X suivant l'une des revendications précédentes, **caractérisé en ce qu'**il a, en outre, au moins un générateur monté fixe et/ou au moins un dispositif de refroidissement monté fixe ayant des connexions (9) pour le au moins un dispositif (2a, 2b, 16a, 16b, 49) à rayons X mobile.

10. Système (1a, 1b, 1c, 1d, 1e, 1f) à rayons X suivant l'une des revendications précédentes, **caractérisé en ce qu'**au moins l'un des dispositifs (2a, 2b, 16a, 16b, 48, 49) à rayons X, notamment un dispositif (2a, 2b, 48) à rayons X monté fixe, a un système (12) d'image, dans lequel au moins un autre des dispositifs (2a, 2b, 16a, 16b, 48, 49) à rayons X, notamment un dispositif (2a, 2b, 16a, 16b, 49) à rayons X mobile, est constitué pour être utilisé avec le système (12) d'image, notamment par transmission de données d'image par la liaison (3) de communication.

11. Système (1a, 1b, 1c, 1d, 1e, 1f) à rayons X suivant la revendication 10, **caractérisé en ce que** le système (12) d'image est constitué pour l'exploitation commune de données d'image de plusieurs dispositifs (2a, 2b, 16a, 16b, 48, 49) à rayons X.

12. Système (1a, 1b, 1c, 1d, 1e, 1f) à rayons X suivant l'une des revendications précédentes, **caractérisé en ce que** le système (1a, 1b, 1c, 1d, 1e, 1f) à rayons X a au moins un dispositif (10) de représentation d'ensembles de données d'image enregistrés par les dispositifs (2a, 2b, 16a, 16b, 48, 49) à rayons X.

13. Système (1a, 1b, 1c, 1d, 1e, 1f) à rayons X suivant la revendication 12, **caractérisé en ce que** le dispositif (10) de représentation est constitué pour la représentation, sous forme d'ensembles de données d'image, conjointement dans une visualisation en 3D à la manière d'un livre (41), d'images (39, 40) de projection en deux dimensions enregistrées notamment sous des angles de projection différents, notamment par des dispositifs (2a, 2b, 16a, 16b, 48, 49) à rayons X différents, chaque page (42) représentée du livre (41) correspondant à une image (39, 40) de projection et les pages (42) faisant entre elles un angle (43) qui correspond à la différence d'angle de projection des images (39, 40) de projection respectives.

14. Système (1a, 1b, 1c, 1d, 1e, 1f) à rayons X suivant l'une des revendications précédentes, **caractérisé en ce que** l'un des dispositifs (2a, 2b, 16a, 16b, 48, 49) à rayons X est un dispositif (48) à rayons X monté fixe et suspendu par le couvercle ayant un arceau (20) en C qui porte le système d'enregistrement ou un dispositif Sliding Gantry-CT, dont les dispositifs (11, 10) de service et/ou de représentation sont, sur la base de la au moins une liaison (3) de communication, constitués également pour être utilisés avec le au moins un dispositif (2a, 2b, 16a, 16b, 49) à rayons X mobile.

15. Procédé pour faire fonctionner un système (1a, 1b, 1c, 1d, 1e, 1f) à rayons X, comprenant au moins deux dispositifs (2a, 2b, 16a, 16b, 48, 49) à rayons X, ayant chacun un dispositif (5) de commande et un système d'enregistrement ayant un émetteur (21) de rayons X et un détecteur (22) de rayons X, dans lequel au moins l'un des dispositifs (2a, 2b, 16a, 16b, 48, 49) à rayons X est un dispositif (2a, 2b, 16a, 16b, 49) à rayons X mobile ayant un support (19) mobile portant le système d'enregistrement, dans lequel au moyen de, respectivement, une interface (4) de communication des dispositifs (2a, 2b, 16a, 16b, 48, 49) à rayons X, on établit une liaison (3) de communication entre les dispositifs (5) de commande des dispositifs (2a, 2b, 16a, 16b, 48, 49) à rayons X et/ou entre un dispositif (7) intermédiaire appartenant au système (1a, 1b, 1c, 1d, 1e, 1f) à rayons X, qui a soi-même un dispositif (5) de commande, et au moins une interface (8) de communication et tous les dispositifs (5) de commande des dispositifs (2a, 2b, 16a, 16b, 48, 49) à rayons X, le dispositif (5) de commande d'au moins l'un des au moins un dispositif (2a, 2b, 16a, 16b, 49) à rayons X mobile commandant le fonctionnement d'enregistrement d'image du dispositif (2a, 2b, 16a, 16b, 48, 49) à rayons X respectif à l'aide de données de commande, obtenues d'un autre dispositif (5) de commande par la liaison (3) de communication, et/ou transmettant au moins un ensemble de données d'image enregistré à au moins un autre dispositif (5) de commande par la liaison (3) de communication.
